# EUROPEAN PATENT APPLICATION

(11) **EP 4 790 064 A1**
(43) Date of publication of application: **12.08.2026**
(21) Application number: 25382110.2
(22) Date of filing: 10.02.2025
(51) Int. Cl.: C07K 5/107, C07K 5/10, A61K 38/05, A61P 1/04, C07K 5/087, C07K 5/08

(54) **CASPASE-1 INHIBITOR COMPOUNDS**

(71) Applicant: Kintsugi Therapeutics S.L., 08018 Barcelona (ES)
(72) Inventor: CUSACHS FARRARÓ, Marc, 08018 Barcelona (ES); HERRERO MOLINA, Carmen, 08018 Barcelona (ES)
(74) Representative: Hoffmann Eitle

(57) **Abstract**

The invention relates to compounds of formula (I): 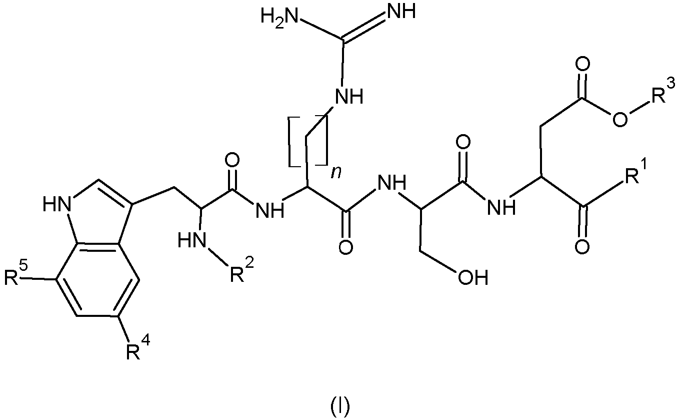 or pharmaceutically acceptable salts, solvates or stereoisomers thereof, to pharmaceutical compositions comprising them and to their use in the treatment and/or prevention of diseases or disorders mediated by Caspase-1.

## Description

### FIELD OF THE INVENTION

The present invention relates to new peptide derivatives, pharmaceutical compositions comprising them and their use in the prevention and/or treatment of Caspase-1 mediated diseases or disorders.

### STATE OF THE ART

Caspases are a family of evolutionary conserved cysteine-dependent endoproteases that hydrolyze their substrates after specific aspartic acid residues (Lamkanfi, 2002). Caspases have been associated with a wide range of biological activities such as apoptosis (Ramirez and Salvesen, 2018), inflammation (Vande Walle, 2016), cell differentiation (Fernando, 2002) and metabolism (Shalini, 2015).

Caspases are classified in two major groups: those involved in the regulation of inflammatory processes (-1, -4, -5, -11, -12) and those that are central to the initiation and execution of apoptosis (2, -3, -7, -8, -9, -10) (Shalini, 2015).

Caspases are cysteine proteases with strict specificity for cleaving peptide sequences C-terminal to aspartic acids residues. Currently, 12 caspase isozymes have been identified in humans, with numerous reported activities. Caspases are often subcategorized as either pro-apoptotic or pro-inflammatory enzymes. A prominent member of the pro-inflammatory class is caspase 1, also known as interleukin-converting enzyme or ICE, which is responsible for the proteolytic activation of interleukin (IL)-1β and IL-18. IL-1β and IL-18 are cytokines that play a major role in the immune response and within numerous autoimmune and inflammatory diseases. Caspase 1 is constitutively and inducibly expressed in immune response elements such as T cells, macrophages and neutrophils. Procaspase 1 is known to associate with several multi-protein complexes capable of responding to numerous external stimuli, suggesting that caspase 1 is a major regulator of the inflammation response. Targeting proteases, and specifically caspases, via small molecule therapeutics is an active area of research. Inhibitors of caspase 1 are sought for intervention strategies within inflammation, inflammasome, pyroptosis and fibrosis mediated diseases such as:
- Autoinflammatory diseases including cryopyrin-associated periodic syndromes, Muckle-Wells syndrome, Schnitzler's syndrome, familial Mediterranean fever, macrophage activation syndrome, Crohn's disease, ulcerative colitis, and inflammatory bowel disease.
- Ischemic diseases and injuries including atherosclerosis, myocardial infarction, heart ischemia, kidney ischemia, neonatal brain ischemia, hypoxia-ischemia injuries, stroke-like brain injuries, and ischemic optic neuropathy.
- Neurodegenerative and neurological diseases including Alzheimer's disease, Parkinson's disease, Huntington's disease, mild cognitive impairment, amyotrophic lateral sclerosis (ALS), multiple sclerosis, multiple system atrophy, epilepsy, Creutzfeldt-Jakob disease, and adrenoleukodystrophy.
- Metabolic and endocrine diseases including type 2 diabetes, dyslipidemias, obesity, metabolic syndrome, metabolic-dysfunction-associated fatty liver disease (MAFLD), metabolic dysfunction-associated steatohepatitis (MASH), cirrhosis, autoimmune hepatitis, primary sclerosing cholangitis and alpha-1 antitrypsin deficiency.
- Inflammatory and autoimmune diseases including osteoarthritis, rheumatoid arthritis, psoriatic arthritis, gout, lupus nephritis, Behçet disease, and sterile inflammatory conditions such as syndromes triggered by aluminum salts.
- Pulmonary diseases including steroid-resistant asthma, chronic obstructive pulmonary disease (COPD), idiopathic pulmonary fibrosis, and acute lung injury.
- Ophthalmic diseases including retinal detachment, macular degeneration, diabetic retinopathy, retinitis pigmentosa, blunt ocular injury, glaucoma, and non-arteritic ischemic optic neuropathy.
- Infectious and immune-related diseases including bacterial infections (e.g., Brucella, Staphylococcus aureus, and Salmonella), viral infections (e.g., influenza, Maraba virus, vesicular stomatitis virus), and sepsis.
- Skin and sensory conditions including inflammatory acne, plaque psoriasis, dermatitis, cytotoxicity-related skin damage, age-related hearing loss, and drug-induced hearing loss.
- Oncological diseases including cancers such as non-small cell lung cancer, breast cancer, melanoma, renal cell carcinoma, and pancreatic cancer.
- Other conditions including peritonitis, polycystic kidney disease, acute kidney injury, joint loosening, and traumatic brain injury.

The overall goal that lead to this invention was to screen for and/or design inhibitors of caspase 1 that were potent and selective over the additional caspase isozymes. The compounds of the invention aim to meet these needs.

### BRIEF DESCRIPTION OF THE FIGURES

**Figure 1****.** Inhibitor potency (*k_{obs}*/*l) profiles for caspase-1,-3,-6,-7,-8,-9 and -10.*
**Figure 2****.** ADME Lead optimization sublibrary from sequence Trp-Arg-Ser-Asp.
**Figure 3****.** Inhibitor potency (k*_{obs}*/*l*) profiles for caspase-1.
**Figure 4****.** Cellular permeability *(Papp (10⁻⁶ cm*/*sec*)) profiles for inhibitors.
**Figure 5****.** Cellular death (expressed as increase from vehicle) for inhibitors.
**Figure 6****.** Accumulation of lipids (expressed as increase from vehicle).
**Figure 7****. Production of IL-1b (expressed as increase from control).**
**Figure 8****. Production of IL-18 (expressed as increase from control).**
**Figure 9****. In vivo model of peritonitis and inflammasome activation.**
**Figure 10****. In vivo MASH: ALT, eWAT weight and fibrosis**
**Figure 11****. In vivo model of MASH: Liver histology**
**Figure 12****. In vivo model of ulcerative colitis**

### DETAILED DESCRIPTION OF THE INVENTION

In the context of the present invention, the following terms have the meaning detailed below:
The term "C₁₋₆ alkyl" refers to a linear or branched hydrocarbon chain radical consisting of carbon and hydrogen atoms, containing no unsaturation, having between 1 and 6, preferably between 1 and 3 ("C₁₋₃ alkyl"), more preferably 1 or 2 ("C₁₋₂ alkyl"), carbon atoms and which is attached to the rest of the molecule by a single bond, including for example and in a non-limiting sense, methyl, ethyl, n-propyl, i-propyl, n-butyl, t-butyl, n-pentyl, etc. Preferably "alkyl" refers to methyl or ethyl.

The term "C₃₋₇ cycloalkyl" refers to a saturated or partially saturated mono- or bicyclic aliphatic group having between 3 and 7, preferably between 3 and 6 carbon atoms which is bound to the rest of the molecule by means of a single bond, including for example and in a non-limiting sense, cyclopropyl, cyclohexyl or cyclopentyl.

The term "C₆₋₁₀ aryl" refers to an aromatic group having between 6 and 10, preferably 6 or 10 carbon atoms, comprising 1 or 2 aromatic nuclei, including for example and in a non-limiting sense, phenyl, naphthyl, etc. Preferably "aryl" refers to phenyl.

The term "halogen" refers to bromo, chloro, iodo or fluoro.

The term "C₁-C₆ haloalkyl" refers to an alkyl group as defined above wherein at least one of the hydrogen atoms has been replaced by a halogen atom such as, for example CF₃, CCl₃, CHF₂, CH₂F, CF₂CF₃.

The term "C₁₋₆ alkoxyl" refers to a group of formula -O-C₁₋₆ alkyl, wherein C₁₋₆ alkyl is an alkyl group as defined above, preferably C₁₋₃ alkyl. Examples of C₁₋₆ alkoxyl include methoxy, ethoxy, propoxy, iso-propoxy, butoxy, tert-butoxy, iso-butoxy and sec-butoxy, preferably methoxy.

The term "(C₆-C₁₀)aryl(C₁-C₆)alkyl" refers to an aryl group as defined above which is attached to the rest of the molecule through an alkyl group as defined above. Preferably, the (C₆-C₁₀)aryl(C₁-C₆)alkyl is a (C₆)aryl(C₁-C₃)alkyl, such as benzyl.

"5- to 10-membered heterocyclyl" refers to a stable 5- to 10-membered ring radical, preferably a 5- or 6-membered ring, which consists of carbon atoms and from one to five, preferably from 1 to 4, heteroatoms selected from the group consisting of nitrogen, oxygen, and sulphur and which can be partially or fully saturated. For the purposes of this invention, the heterocycle can be a monocyclyl or bicyclyl ring system. Examples of such heterocycles include, but are not limited to, pyrrolidine, piperidine, tetrahydropyridine, piperazine, morpholine, thiomorpholine, diazepane, tetrahydrofuran, tetrahydropyran, octahydro-pyrrolopyrazine.

"5- to 10-membered heteroaryl" refers to a stable 5- to 10-membered aromatic ring radical, preferably a 5- or 6-membered aromatic ring, which consists of carbon atoms and from one to five, preferably from 1 to 4, heteroatoms selected from the group consisting of nitrogen, oxygen, and sulphur. For the purposes of this invention, the heteroaryl can be a monocyclyl or bicyclyl ring system. Examples of such heteroaryl include, but are not limited to, thiophene, furan, pyrrole, thiazole, oxazole, isothiazole, isooxazole, imidazole, pyrazole, triazole, oxadiazole, thiadiazole, tetrazole, tetrazole oxide, oxadiazolone, pyridine, pyrimidine, dihydroindolone, benzimidazole, benzothiazole, benzofuran, indole, purine, quinoline.

As understood in this technical area, there can be a certain degree of substitution on the previously defined radicals. Thus, there can be substitution in any of the groups of the present invention. The references of the present document to substituted groups in the groups of the present invention indicate that the specified radical can be substituted in one or more available positions by one or more substituents. Said substituents include, for example and in a non-limiting sense, halogen, C₁₋₆ alkyl, C₃₋₇ cycloalkyl, C₁₋₆ haloalkyl, C₆₋₁₀ aryl, (C₆₋₁₀)aryl(C₁₋₆)alkyl, 5- to 10-membered heterocyclyl, 5- to 10-membered heteroaryl, -CN, -NO₂, -OR, -SR, -C(O)R, -C(O)OR, -OC(O)R, - C(O)NR₂, -NR₂ and -SO₂R; wherein each R is independently selected from H, C₁₋₆ alkyl, C₁₋₆ haloalkyl, C₃₋₇ cycloalkyl, C₆₋₁₀ aryl, (C₆₋₁₀)aryl(C₁₋₆)alkyl, 5- to 10-membered heterocyclyl, and 5- to 10-membered heteroaryl.

The compounds of the invention may be in the form of salts, solvates or stereoisomers, preferably pharmaceutically acceptable salts, solvates or stereoisomers.

As already indicated, the present invention also provides "salts" of the compounds described herein. By way of illustration, said salts can be acid addition salts, base addition salts or metal salts, and can be synthesized from the parent compounds containing a basic or acid moiety by means of conventional chemical processes known by the person skilled in the art. See, generally, G. S. Paulekuhn, et al., "Trends in Active Pharmaceutical Ingredient Salt Selection based on Analysis of the Orange Book Database", J. Med. Chem., 2007, 50: 6665-72, S. M. Berge, et al., "Pharmaceutical Salts", J Pharm Sci., 1977, 66:1-19, and Handbook of Pharmaceutical Salts, Properties, Selection, and Use, Stahl and Wermuth, Eds., Wiley-VCH and VHCA, Zurich, 2002. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, pyrosulfate, bisulfate, sulfite, bisulfite, nitrate, phosphate, monohydrogen-phosphate, dihydrogenphosphate, meta-phosphate, pyrophosphate, etc., organic acid addition salts such as, for example, acetate, maleate, fumarate, citrate, oxalate, succinate, tartrate, malate, mandelate, methanesulfonate, p-toluenesulfonate, camphorsulfonate, propionates, decanoates, caprylates, acrylates, formates, isobutyrates, caproates, heptanoates, propiolates, malonates, succinates, suberates, sebacates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, lactates, y-hydroxybutyrates, glycolates, propanesulfonates, naphthalene-1-sulfonates, naphthalene-2-sulfonates, etc. Illustrative examples of base addition salts include inorganic base salts such as, for example, ammonium salts and organic base salts such as, for example, ethylenediamine, ethanolamine, N,N-dialkylenethanolamine, triethanolamine, glutamine, amino acid basic salts, etc. Illustrative examples of metal salts include, for example, sodium, potassium, calcium, magnesium, aluminum and lithium salts.

The term "solvate" according to this invention is to be understood as meaning any form of the active compound according to the invention which has another molecule (most likely a polar solvent) attached to it via non-covalent bonding. Examples of solvates include hydrates and alcoholates. Solvation methods are generally known in the state of the art.

As used herein, the term "stereoisomer" is a general term for all isomers of individual molecules that differ only in the orientation of their atoms in space. Therefore, stereoisomer compounds are molecules that are non-superimposable mirror images of each other and include enantiomers and diastereomers.

The term "chiral center" refers to a carbon atom to which four different groups are attached.

The terms "enantiomer" and "enantiomeric form" refer to one of the two stereoisomers of a compound that are mirror images of each other that are non-superimposable. Enantiomer compounds are optically active, wherein one enantiomer rotates the plane of polarized light in one direction and the other one rotates the plane of polarized light in the opposite direction and form a racemic compound when present in equal quantities.

The term "racemic" or "racemate" refers to a mixture that has equal amounts of enantiomers and which mixture is optically inactive.

The terms "diastereomer" and "diastereomeric form" refer to stereoisomers of a compound with more than one chiral center that are not mirror images of one another.

As will be understood, the terms enantiomerically enriched or diastereomerically enriched describes a mixture of two enantiomers or a mixture of diastereomers in which one enantiomer or diastereomer is present in greater amount than the other enantiomer or diastereomer.

The compounds of the invention have chiral centers and therefore can exist in different stereoisomeric forms, such as enantiomeric or diastereomeric forms. Thus, any given compound referred to herein is intended to represent any one of a racemate, one or more enantiomeric forms and one or more diastereomeric forms. All the stereoisomers including enantiomers and diastereoisomers of the compounds referred to herein, and mixtures thereof (including racemic mixtures, enantiomerically enriched mixtures and diastereomerically enriched mixtures), are considered within the scope of the present invention.

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans.

A "fluorophore", as used herein refers to a molecule or moiety that can re-emit light upon light excitation.

As used herein, the term "warhead" refers to a moiety present on a compound of the present invention which is capable of covalently binding with an active site of the target enzyme (Caspase-1), thereby achieving irreversible inhibition effects. One of ordinary skill in the art will recognize that certain reactive functional groups can act as warheads. It is noted that "Covalently binding" is not equal to "irreversible inhibition". For example aldehydes bind covalently but reversibly. Even some AOMKs can act as reversible inhibitors:
1) Brady KD. Bimodal inhibition of caspase-1 by aryloxymethyl and acyloxymethyl ketones. Biochemistry. 1998; 37:8508-15.
2) Brady KD, Giegel DA, Grinnell C, Lunney E, Talanian RV, Wong W, et al. A catalytic mechanism for caspase-1 and for bimodal inhibition of caspase-1 by activated aspartic ketones. Bioorg Med Chem. 1999; 7:621-31.

Poreba et al. Caspase selective reagents for diagnosing apoptotic mechanisms, CDD 2019.

As used herein the expression "prodrug" refers to compounds that are drug precursors which following administration, release the drug (or "active") *in vivo* via some chemical or physiological process (e.g., hydrolysis, enzymatic cleavage or hydrolysis, or metabolism is converted to the desired drug form). The invention includes within its scope prodrugs such as those compounds of the invention methylated in P1 (Asp), wherein *in vivo* demethylation of the P1 group shall release the active ingredient.

### Compounds of the invention

The present specification exemplifies potent and selective caspase-1 ligands. In particular, the present application exemplifies compounds of formula (I): 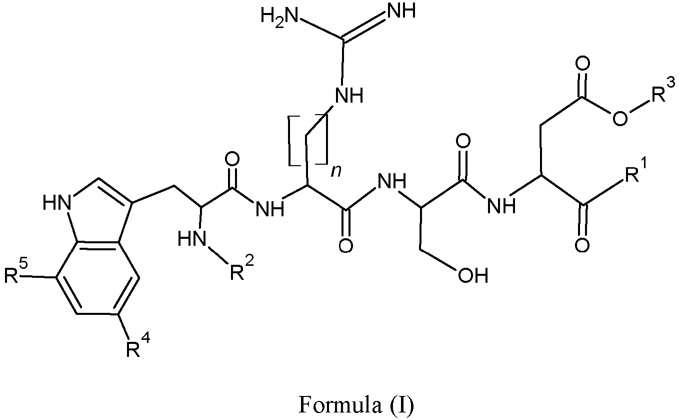 or a salt, solvate or stereoisomer thereof;
wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen;
R₄, and R₅ are each independently selected from a hydrogen, a halogen or a C₁₋₄alkyl including branched alkyl and cycloalkyl, wherein each C₁₋₄ alkyl is optionally substituted with 1 -3 halogen atoms;
n is an integer between 1 and 3; and
R₁ is a chemically reactive group such as a fluorophore or a warhead.

In particular, the present invention discloses specific ligands of formula (I) with fluorine substituents. It is noted that decoration of organic molecules with fluorine substituents is a widely used strategy to improve small molecule drugs, owing to fluorine's capacity to productively affect molecular properties, such as conformation, pKa values of neighboring functional groups, and hydrophobicity. Often, this leads to improved interactions with proteins, enzymes, receptors, or membranes, thereby improving pharmacological properties, cell-permeability, bioavailability, and activity (S. Purser, Chem. Soc. Rev. 2008, 37, 320-330.). Moreover, due to the remarkable stability of the fluorine covalent bond, these molecules often show improved metabolic stability.

With the advent of easy to access fluorine-containing amino acids (J. Moschner, Chem. Rev. 2019, 119, 10718-10801; J. Leppkes, J. Fluorine Chem. 2020, 232, 109453.), these building blocks sparked a renewed interest for drug design (H. Mei, Eur. J. Med. Chem. 2020, 186, 111826.) and peptide and protein engineering. Selective introduction of fluorine-containing amino acids into peptides can modulate distinct pharmacokinetic and physicochemical functionalities of the resulting biopolymers, including structure, folding or stability towards thermal and chemical denaturation. Furthermore, it can improve metabolic stability, biological activity, cell-permeability and bioavailability of peptide-based drugs (G. Akcay, J. Fluorine Chem. 2009, 130, 1178-1182; M. Salwiczek, Chem. Soc. Rev. 2012, 41, 2135-2171; E. N. G. Marsh, Acc. Chem. Res. 2014, 47, 2878-2886; A. A. Berger, Acc. Chem. Res. 2017, 50, 2093-2103; S. Huhmann, Eur. J. Org. Chem. 2018, 3667-3679; R. Smits, Curr. Top. Med. Chem. 2006, 6, 1483-1498).

In this patent we sought to introduce fluorinated amino acid analogues and methylations in the carboxy groups of the amino acid side chains into a compound that targets caspase-1 to improve its druggability properties.

Therefore, in a first aspect of the present invention we herein provide compounds of general formula (I): 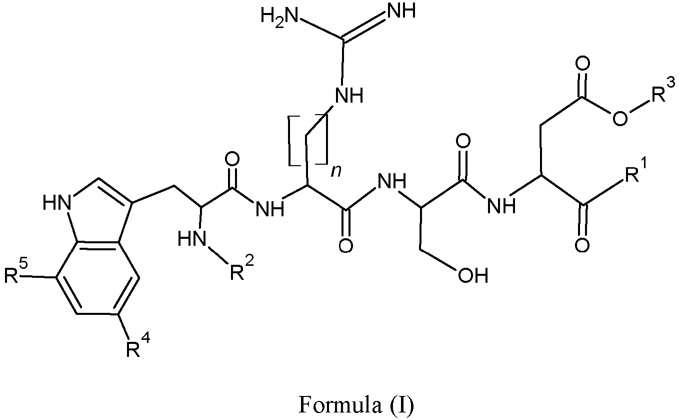 or a salt, solvate or stereoisomer thereof;
wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen;
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms;
n is an integer between 1 and 3; and
R₁ is a chemically reactive group such as a fluorophore or a warhead.

Preferably:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen;;
n is 3;
R₄, and R₅ are each independently selected from a hydrogen, or a halogen atom; and
R₁ is a chemically reactive group.

In a preferred embodiment of the first aspect of the invention, the compound is of formula (Ia): 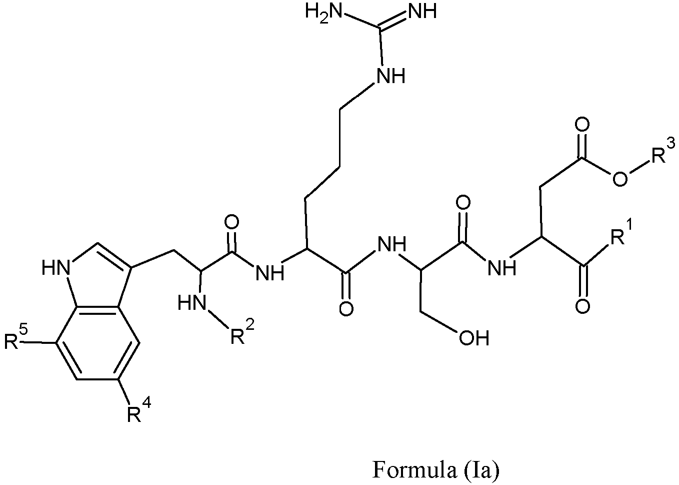 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen;
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms; and
R₁ is a chemically reactive group.

Preferably:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen;
R₄, and R₅ are each independently selected from a hydrogen, or a halogen atom;
R₁ is a chemically reactive group.

In a preferred embodiment of the first aspect of the invention, the compound is of formula (II): 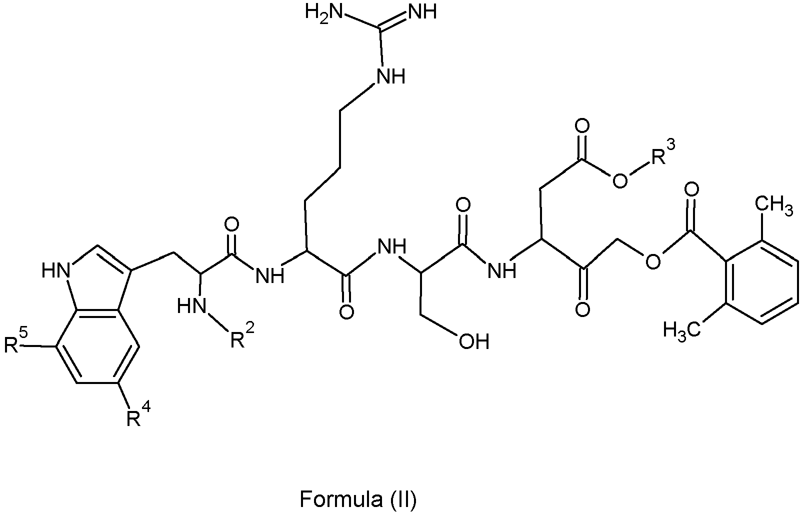 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms.

Preferably:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom.

In a preferred embodiment of the first aspect of the invention, the compound is of formula (III): 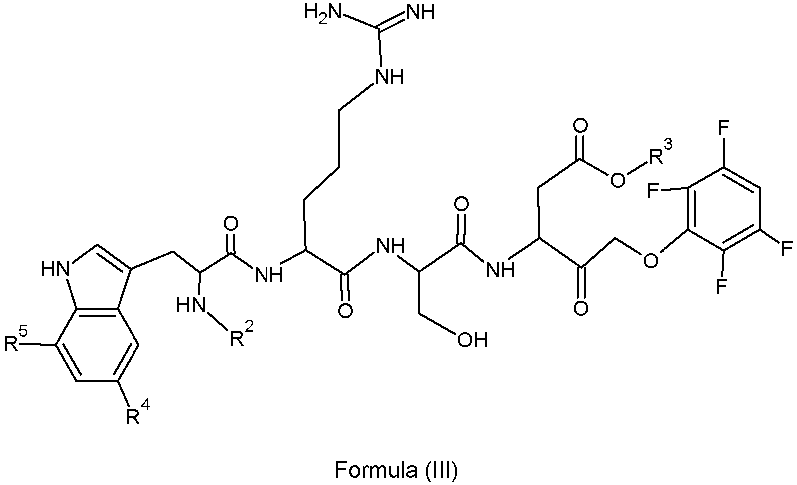 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms.

Preferably:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom.

In a preferred embodiment of the first aspect of the invention, the compound is of formula (IV): 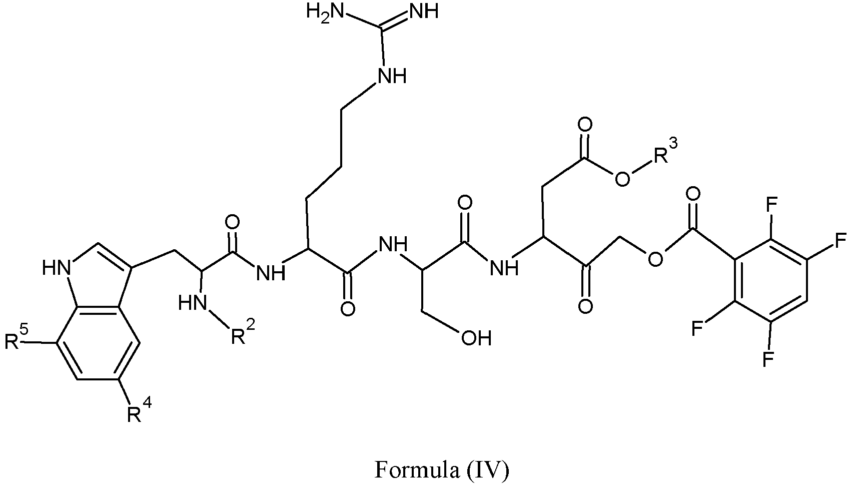 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms.

Preferably:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom.

In another preferred embodiment of the first aspect of the invention, the compound is selected from any one of the following chemical compounds (protonated or deprotonated) or a salt, solvate or stereoisomer thereof:
a. 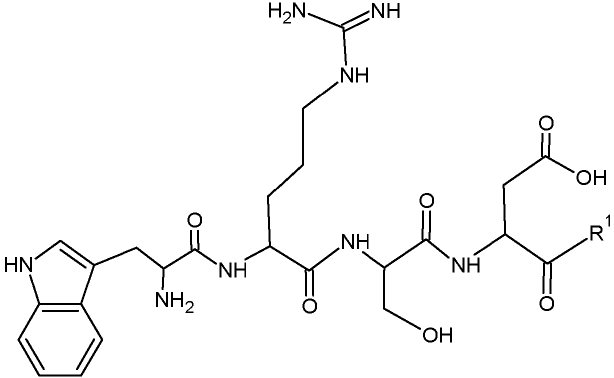
b. 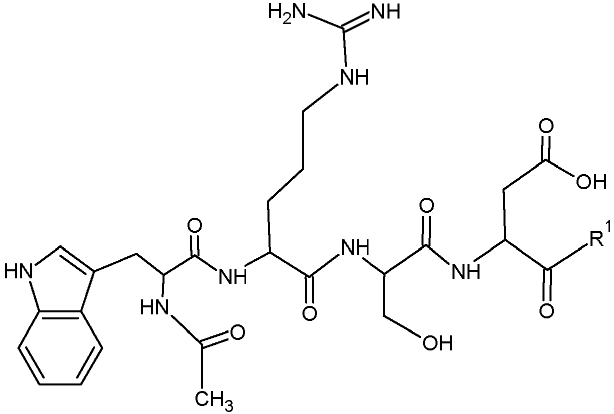
c. 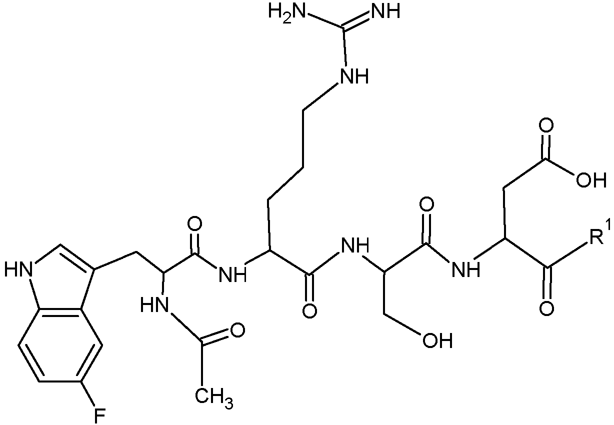
d. 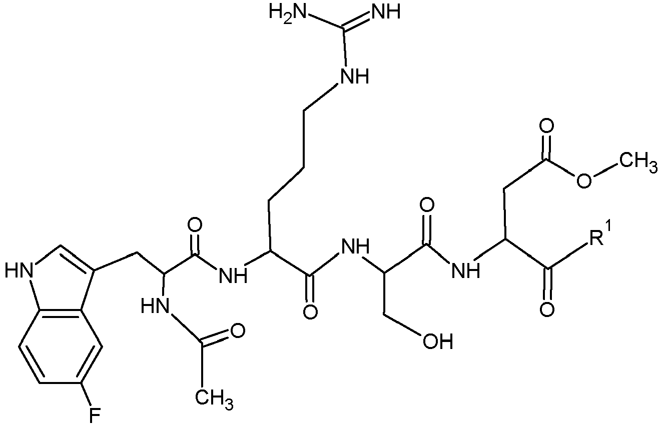
e. 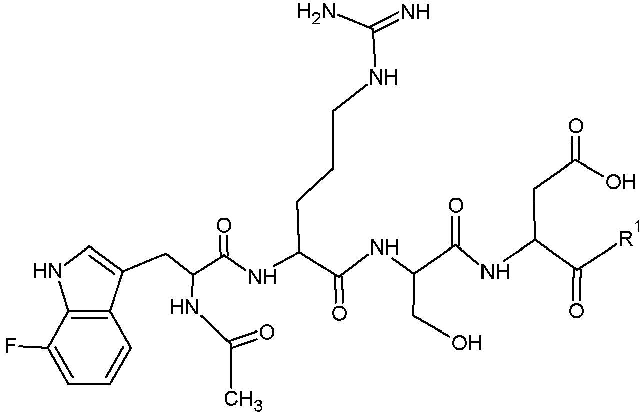
f. 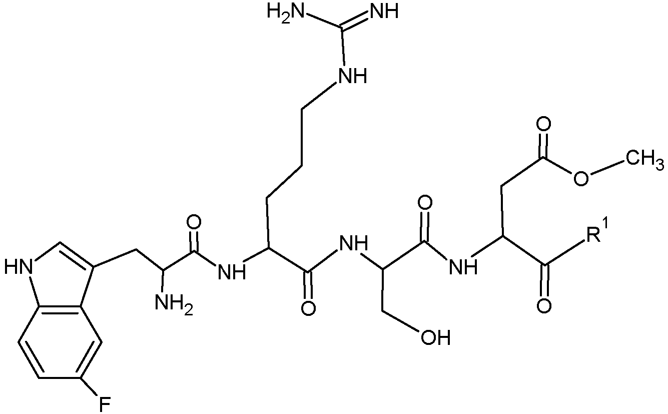
g. 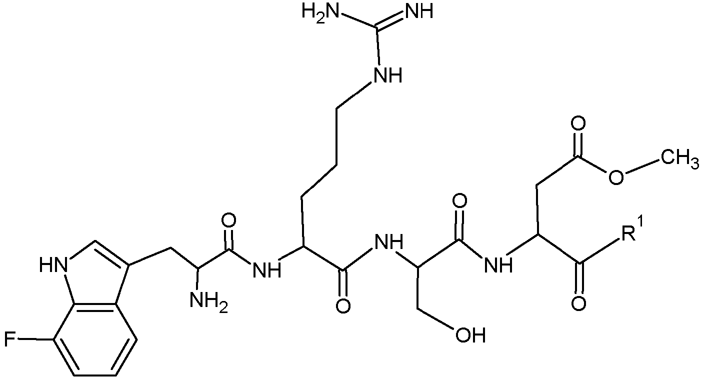
h.

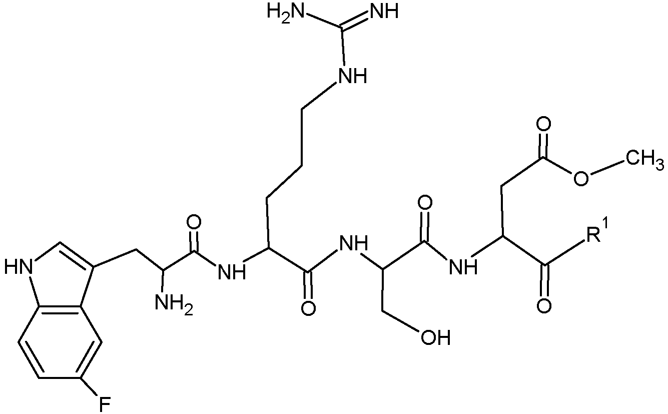

Notwithstanding the above, in another preferred embodiment of the first aspect of the invention, a compound of the invention is selected from any one of the following chemical compounds (protonated or deprotonated) or a salt, solvate or stereoisomer thereof:
1. Trp-Glu-Ala-Asp
2. Trp-Glu-His-Asp
3. Phe(4-I)-Glu-Thr-Asp
4. Trp-Arg-Ser-Asp
5. Phe(4-I)-Glu-Ile-Asp
6. Cha-Glu-Aoc-Asp
7. Leu-Tle-Ala-Asp
8. hLeu-Glu-Ala-Asp
9. Leu-Abu-hCha-Asp
10. Leu-Glu-hCha-Asp
11. Trp-Lys-Glu-Asp
12. Nle-hGlu-hPhe-Asp
13. hLeu-Tle-Ser-Asp
14. Tyr-Lys-Ala-Asp
15. Phe(4-Me)-hGlu-Tle-Asp
16. Nle-hGlu-Pro-Asp
17. Leu-Tle-His-Asp
18. Nle-Glu-Ser-Asp
19. Leu-Glu-His-Asp
20. Phe(NH2)-hGlu-Cha-Asp
21. Tle-hGlu-hCha-Asp
22. Tyr-Thz-Ala-Asp
23. hleu-hGlu-Ser-Asp
24. Asp-Leu-Thr-Ala-Asp
25. Chg-hGlu-hCha-Asp
26. hPro-Tle-Ser-Asp
27. Leu-Gln-Ser-Asp
28. Leu-Phe-Ser-Asp
29. Oic-Leu-Ala-Asp
30. Pro-Gln-Ala-Asp
31. hPro-Leu-Ala-Asp
32. Lys(Ac)-hGlu-Ala-Asp
33. hPro-Val-Ser-Asp
34. hPhe-hGlu-hPro-Asp

wherein compounds 1 to 34 above are further characterized by being substrates and having a R1 group linked to the P1 subunit through a covalent amide bond between the amino group of the R1 subunit and the α-carboxyl group of the P1 amino acid; or
by being inhibitors and having P1 linked to R1 through a covalent bond between the α-carboxyl group of the P1 amino acid and the R1 moiety, wherein R1 could be a large number of moieties such as acyloxymethyl ketone (in AOMK) or just a simple hydrogen atom (in aldehydes).

Below is a concise set of definitions suitable for a patent specification. Unless otherwise specified, each abbreviation refers to the naturally occurring L-(2S) stereoisomer.
Trp: Refers to tryptophan.
Asp: Refers to aspartic acid.
His: Refers to histidine.
Phe(4-I): Refers to 4-iodophenylalanine.
Thr: Refers to threonine.
Arg: Refers to arginine.
Ser: Refers to serine.
Ile: Refers to isoleucine.
Cha: Refers to cyclohexylalanine.
Aoc: Refers to 8-aminooctanoic acid (H₂N-(CH₂)₇-COOH).
Leu: Refers to leucine, preferably L-leucine.
Abu: Refers to 2-aminobutyric acid, preferably L-2-aminobutyric acid.
Nle: Refers to norleucine, preferably L-norleucine.
hGlu: Refers to homoglutamic acid, preferably L-homoglutamic acid.
hPhe: Refers to homophenylalanine, preferably L-homophenylalanine.
hLeu: Refers to homoleucine, preferably L-homoleucine.
Ala: Refers to alanine, preferably L-alanine.
Tle: Refers to tert-leucine, preferably L-tert-leucine.
Tyr: Refers to tyrosine.
Lys: Refers to lysine.
Pro: Refers to proline.
Thz: Refers to thiazolidine-4-carboxylic acid, a cyclic analog of cysteine/proline.
Chg: Refers to cyclohexylglycine.
hCha: Refers to homocyclohexylalanine, an extended homolog of cyclohexylalanine.
hPro: Refers to homoproline.
Oic: Refers to octahydroindole-2-carboxylic acid, a bicyclic proline analog.
Gln: Refers to glutamine.
Lys(Ac): Refers lysine bearing an acetyl substituent on the side-chain amino group.
Glu: Refers to glutamic acid.

For clarity, where "h" appears in an abbreviation (e.g., hLeu), it denotes a "homo" extension by one additional methylene unit compared to the parent side chain. All references to these amino acids in this specification, including the claims, are preferably intended to denote their L-stereochemistry unless otherwise stated.

In this sense it is noted that for all of substrates provided throughout the present specification (such as those comprising the fluorophores), R1 is linked to the P1 subunit through a covalent amide bond between the amino group of the R1 subunit (or any other group indicated as W) and the α-carboxyl group of the P1 amino acid. Preferably, R1 is a moiety such as a fluorophore as ACC of formula:

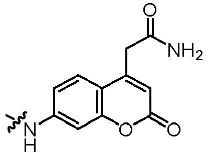

More particularly, R₁ can be a moiety such as a fluorophore (to be use in the context of substrates) selected from a group of formula: 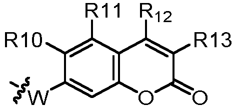 wherein
W is selected from -NH- and -O-,
R10-R13 are independently selected from H, C₁₋₆ alkyl and C₃₋₇ cycloalkyl, Aryl, heteroaryl, CF₃, -CH₂COOH, -CH₂CONHR14, and CH₂OR14, and R14 is selected from H, C1-6 alkyl and C3-7 cycloalkyl.

Examples of R1 moieties as fluorophores can be selected from any of the following list:

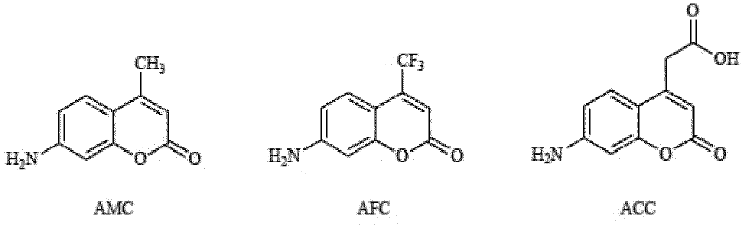

Other examples of potentially useful moieties as fluorophores can be selected from the list consisting of: 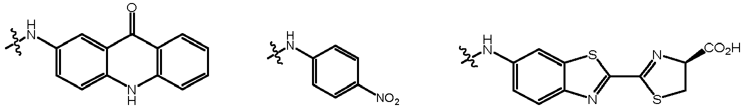 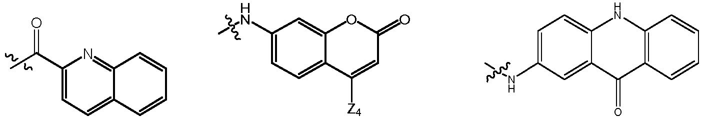 or

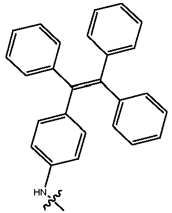

In the context of the present invention, a fluorophore (or fluorochrome, similarly to a chromophore) is a fluorescent chemical compound that can re-emit light upon light excitation. Fluorophores or substrates typically contain several combined aromatic groups, or planar or cyclic molecules with several π bonds. Fluorophores are notably used to stain tissues, cells, or materials in a variety of analytical methods, i.e., fluorescent imaging and spectroscopy. wherein W of the above formula should represent a -NH-such as in ACC. Further fluorophores in R₁ can be selected from common fluorophores including Indo-1, Ca saturated Indo-1 Ca2+ Cascade Blue BSA pH 7.0 Cascade Blue
LysoTracker Blue Alexa 405 LysoSensor Blue pH 5.0 LysoSensor Blue DyLight 405 DyLight 350 BFP (Blue Fluorescent Protein) Alexa 350
7-Amino-4-methylcoumarin pH 7.0, AminoCoumarin AMCA conjugateCoumarin 7-Hydroxy-4-methylcoumarin7-Hydroxy-4methylcoumarin pH 9.0
6,8-Difluoro-7-hydroxy-4-methylcoumarin pH 9.0 Hoechst 33342 Pacific Blue Hoechst 33258 Hoechst 33258-DNA Pacific Blue antibody conjugate pH 8.0 PO-PRO-1PO-PRO-1-DNA POPO-1 433 nm POPO-1-DNA DAPI-DNA DAPI Marina Blue SYTOX Blue-DNA CFP (Cyan Fluorescent Protein) eCFP (Enhanced Cyan Fluorescent Protein) 1-Anilinonaphthalene-8-sulfonic acid (1,8-ANS) Indo-1, Ca free 1,8-ANS (1-Anilinonaphthalene-8-sulfonic acid)
BO-PRO-1-DNA BOPRO-1 BOBO-1-DNA SYTO 45-DNA
evoglow-Pp1 evoglow-Bs1 evoglow-Bs2 Auramine O DiO LysoSensor Green pH 5.0 Cy 2 LysoSensor Green Fura-2, high Ca Fura-2 Ca2+sup> SYTO 13-DNA YO-PRO-1-DNA YOYO-1-DNA eGFP (Enhanced Green Fluorescent Protein) LysoTracker Green GFP (S65T) BODIPY FL, MeOH Sapphire
BODIPY FL conjugate MitoTracker Green MitoTracker Green FM Fluorescein 0.1 M NaOH Calcein pH 9.0 Fluorescein pH 9.0 Calcein
Fura-2, no Ca Fluo-4 FDA DTAF Fluorescein Fluorescein antibody conjugate pH 8.0 CFDA FITC Alexa Fluor 488 hydrazide-water DyLight 488 5-FAM pH 9.0 FITC antibody conjugate pH 8.0 Alexa 488
Rhodamine 110 Rhodamine 110 pH 7.0 Acridine Orange Alexa Fluor 488 antibody conjugate pH 8.0 BCECF pH 5.5 PicoGreendsDNA quantitation reagent SYBR Green I Rhodaminen Green pH 7.0 CyQUANT GR-DNA
NeuroTrace 500/525, green fluorescent Nissl stain-RNA DansylCadaverine
Rhodol Green antibody conjugate pH 8.0 Fluoro-EmeralNissl Fluorescein dextran pH 8.0 Rhodamine Green 5-(and-6)-Carboxy-2', 7' dichlorofluorescein pH 9.0 DansylCadaverine, MeOH eYFP (Enhanced Yellow Fluorescent Protein)
Oregon Green 488 Oregon Green 488 antibody conjugate pH 8.0 Fluo-3
BCECF pH 9.0 SBFI-Na+ Indo-1, Ca saturated Indo-1 Ca2+ Cascade Blue BSA pH 7.0 Cascade Blue
LysoTracker Blue Alexa 405 LysoSensor Blue pH 5.0 LysoSensor Blue DyLight 405 DyLight 350 BFP (Blue Fluorescent Protein) Alexa 350
7-Amino-4-methylcoumarin pH 7.0, AminoCoumarin AMCA conjugateCoumarin 7-Hydroxy-4-methylcoumarin7-Hydroxy-4methylcoumarin pH 9.0
6,8-Difluoro-7-hydroxy-4-methylcoumarin pH 9.0 Hoechst 33342 Pacific Blue Hoechst 33258 Hoechst 33258-DNA Pacific Blue antibody conjugate pH 8.0 PO-PRO-1PO-PRO-1-DNA POPO-1 433 nm POPO-1-DNA DAPI-DNA DAPI Marina Blue SYTOX Blue-DNA CFP (Cyan Fluorescent Protein) eCFP (Enhanced Cyan Fluorescent Protein) 1-Anilinonaphthalene-8-sulfonic acid (1,8-ANS) Indo-1, Ca free 1,8-ANS (1-Anilinonaphthalene-8-sulfonic acid)
BO-PRO-1-DNA BOPRO-1 BOBO-1-DNA SYTO 45-DNA
evoglow-Pp1 evoglow-Bs1 evoglow-Bs2 Auramine O DiO LysoSensor Green pH 5.0 Cy 2 LysoSensor GreenLysoSensor Yellow pH 9.0 Indo-1, Ca saturated Indo-1 Ca2+ Cascade Blue BSA pH 7.0 Cascade Blue
LysoTracker Blue Alexa 405 LysoSensor Blue pH 5.0 LysoSensor Blue DyLight 405 DyLight 350 BFP (Blue Fluorescent Protein) Alexa 350
7-Amino-4-methylcoumarin pH 7.0, AminoCoumarin AMCA conjugateCoumarin 7-Hydroxy-4-methylcoumarin7-Hydroxy-4methylcoumarin pH 9.0
6,8-Difluoro-7-hydroxy-4-methylcoumarin pH 9.0 Hoechst 33342 Pacific Blue Hoechst 33258 Hoechst 33258-DNA Pacific Blue antibody conjugate pH 8.0 PO-PRO-1PO-PRO-1-DNA POPO-1 433 nm POPO-1-DNA DAPI-DNA DAPI Marina Blue SYTOX Blue-DNA CFP (Cyan Fluorescent Protein) eCFP (Enhanced Cyan Fluorescent Protein) 1-Anilinonaphthalene-8-sulfonic acid (1,8-ANS) Indo-1, Ca free 1,8-ANS (1-Anilinonaphthalene-8-sulfonic acid)
BO-PRO-1-DNA BOPRO-1 BOBO-1-DNA SYTO 45-DNA
evoglow-Pp1 evoglow-Bs1 evoglow-Bs2 Auramine O DiO LysoSensor Green pH 5.0 Cy 2 LysoSensor Green.

On the other hand, in the case of any of the inhibitors described in the present specification, P1 is linked to R1 through a covalent bond between the α-carboxyl group of the P1 amino acid and the R1 moiety, wherein R1 could be a large number of moieties such as acyloxymethyl ketone (in AOMK) or just a simple hydrogen atom (in aldehydes).

Thus, in another embodiment, R1 can be a warhead, in particular, R1 can be a warhead (to be use in the context of inhibitors) selected from the group consisting of: 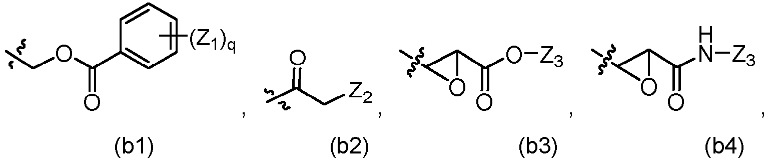 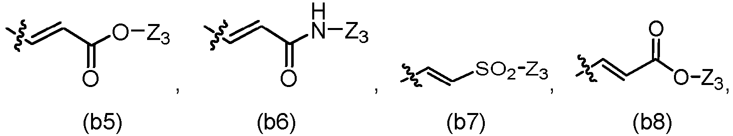 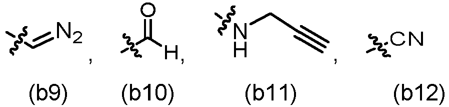 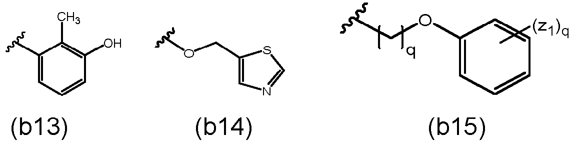 wherein
q is 0, 1, 2, 3, 4 or 5,
each Z₁ is independently selected from C₁₋₆ alkyl, halogen, -CN, -NO₂ and C₁₋₆
alkoxyl,
Z₂ is halogen, and
Z₃ is selected from C₁₋₆ alkyl, C₆₋₁₀ aryl and (C₆₋₁₀)aryl(C₁₋₆)alkyl,
or a salt, solvate or stereoisomer thereof.

Specific examples of warheads useful in the present invention can be selected from any of the following (all of the following compounds are directly bound to the a-COOH group of the P1 subunit):

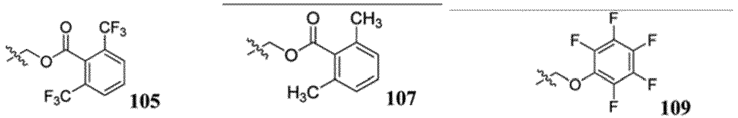

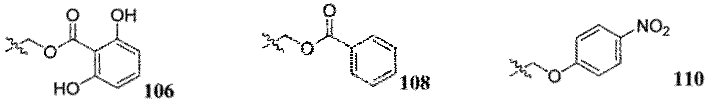

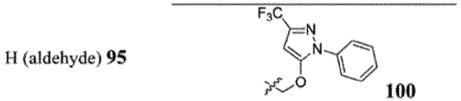

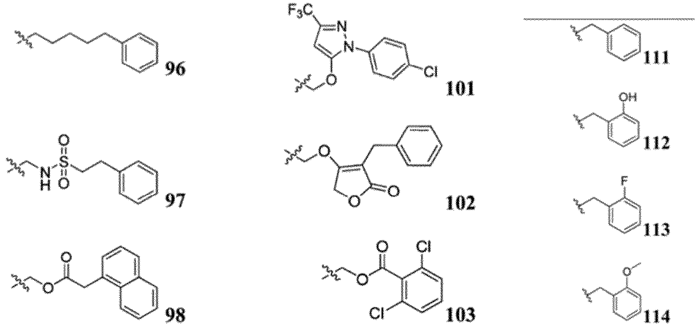

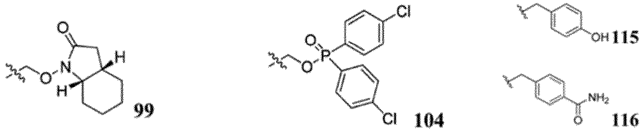

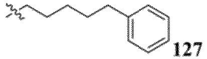

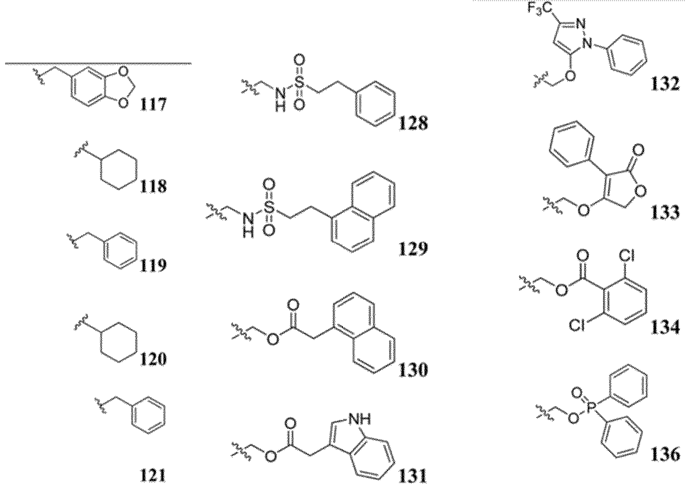

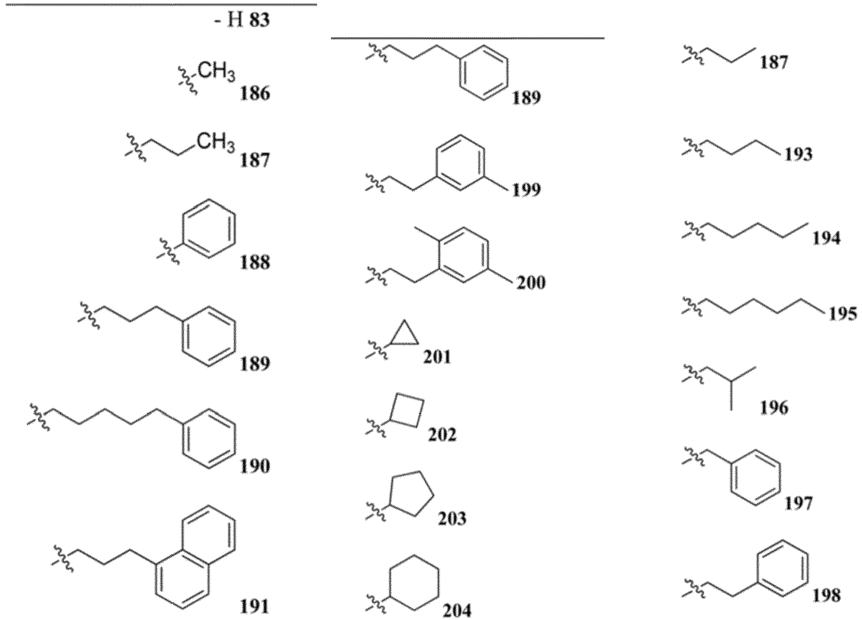

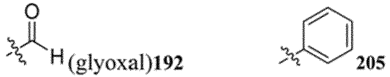

Preferably, R1 is a warhead of formula: 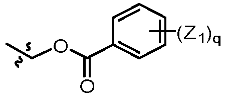 wherein
q is 0, 1, 2, 3 or 4, and
each Z₁ is independently selected from C₁₋₆ alkyl, halogen, -CN and C₁₋₆ alkoxyl.

In an embodiment, Z₁ is independently selected from halogen, methyl and -OMe; preferably from F, Cl and Me (methyl).

In a further embodiment, R₁ is selected from a group of formula 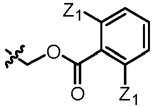 wherein each Z₁ is independently selected from C₁₋₆ alkyl, halogen, -CN and C₁₋₆ alkoxyl; preferably from halogen, methyl and -OMe; more preferably from F, Cl and Me (methyl).

In a preferred embodiment, R₁ has formula:

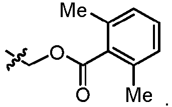

In another preferred embodiment, R₁ is represented by the following formula: 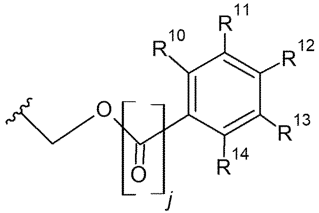 wherein
j is 0 or 1;
R₁₀, R₁₁, R₁₂, R₁₃ and R₁₄ are each independently selected from hydrogen, halogen or a C₁₋₄ alkyl including branched alkyl and cycloalkyl, phenyl, phenoxy, nitro, nitrile, PhCO, NHAc, wherein each alkyl is optionally substituted with 1 -3 halogen atoms.

It is noted that, in certain embodiments, either R10 and R11, R14 and R13, R13 and R12, or R11 and R12 together form an additional aromatic ring, preferably a fused phenyl ring forming a naphthyl group

In another preferred embodiment, R₁ incorporates the P1 group to form a group having the following formula:

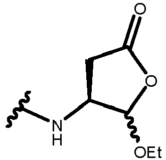

In another embodiment, in the present invention we herein provide any one of the following chemical compounds (protonated or deprotonated) or a salt, solvate or stereoisomer thereof:

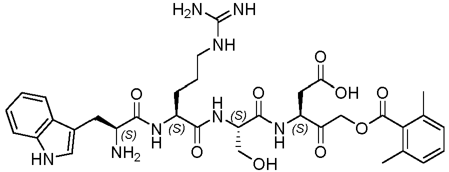

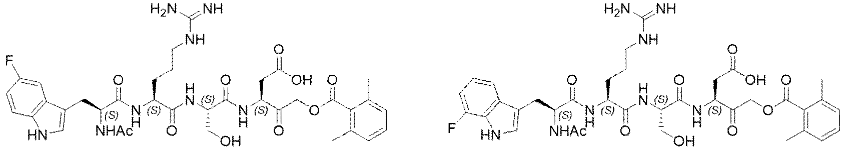

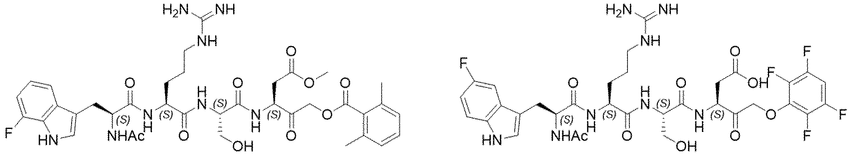

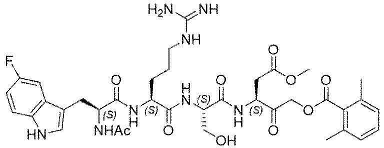

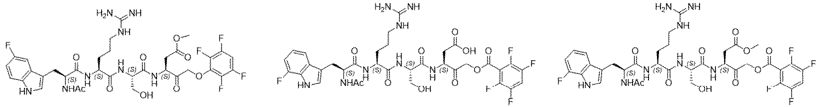

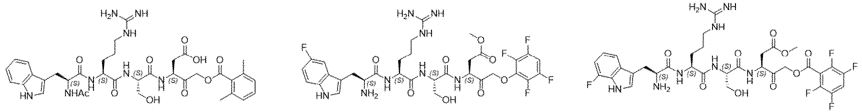

In another embodiment, in the present invention we herein provide the following chemical compound (protonated or deprotonated) or a salt, solvate or stereoisomer thereof:

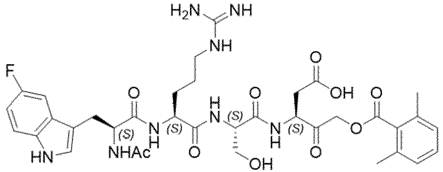

It is noted that all structures above cover all possible forms.

### Pharmaceutical compositions

Compounds of any of the above formulae of the invention where R1 is a warhead such as AOMK, are irreversible inhibitors of Caspase-1 and therefore can be used in the prevention or treatment of disorder or diseases mediates by this enzyme.

Accordingly, in a further aspect the present invention is directed to a pharmaceutical composition comprising a compound of any of the above formulae as defined herein wherein R₁ is a warhead such as AOMK, or a salt, solvate or stereoisomer thereof, and at least one pharmaceutically acceptable excipient.

The term "excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005, or in "Handbook of Pharmaceutical Excipients," Rowe C.R.; Paul J.S.; Marian E.Q., sixth Edition, 2009.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art.

Examples of pharmaceutical compositions include any solid (tablets, pills, capsules, granules etc.) or liquid (solutions, suspensions or emulsions) compositions for oral, topical or parenteral administration.

In an embodiment the pharmaceutical compositions are in oral delivery form. Suitable dose forms for oral administration may be tablets and capsules and may contain conventional excipients known in the art such as binding agents, for example syrup, Arabic gam, gelatin, sorbitol, tragacanth, or polyvinylpyrrolidone; fillers, for example lactose, sugar, corn starch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulfate. Solid oral compositions can be prepared by conventional methods of blending, filling or tabletting. Such operations are conventional in the art. The tablets can be prepared, for example, by dry or wet granulation and optionally can be coated according to methods well known in normal pharmaceutical practice, in particular with an enteric coating.

Pharmaceutical compositions can also be adapted for parenteral administration, such as sterile solutions, suspensions or lyophilized products in the appropriate unit dosage form. Suitable excipients, such as bulking agents, buffering agents or surfactants, can be used.

The mentioned formulations can be prepared using standard methods such as those described or referred to in the European and US Pharmacopoeias and similar reference texts.

The compounds or compositions of the present invention may be administered by any suitable method, such as oral, sublingual, intranasal, intraocular, parenteral, subcutaneous, intramuscular, intravenous, or transdermal administration.

In general, the effective amount of a compound of the invention to be administered will depend on the relative efficacy of the compound chosen, the severity of the disorder being treated and/or prevented and the patient's weight. The active compounds can be administered one or more times a day, for example 1, 2, 3, or 4 times daily, with typical total daily doses in the range from about 0.01 mg/kg of body weight/day to about 1000 mg/kg of body weight/day. In another embodiment, the effective dosage amount of a compounds of the invention is about 500 mg/kg of body weight/day or less. In another embodiment, the effective dosage amount of a compound of the invention is about 100 mg/kg of body weight/day or less. In another embodiment, the effective dosage amount ranges from about 0.01 mg/kg of body weight/day to about 100 mg/kg of body weight/day of a compound of the invention; in another embodiment, from about 0.02 mg/kg of body weight/day to about 50 mg/kg of body weight/day; and in another embodiment, from about 0.025 mg/kg of body weight/day to about 20 mg/kg of body weight/day.

### Uses of the compounds of the invention

Compounds of the invention wherein R₁ is a group comprising a fluorophore moiety can be used as activity-based probes to determine Caspase-1 activity.

Accordingly, in another aspect the invention refers to the *in vitro* use of a compound of the invention as defined herein wherein R₁ is a group comprising a fluorophore moiety, or a salt, solvate or stereoisomer thereof, to determine Caspase-1 activity.

In a particular embodiment, Caspase-1 activity in a cell or a tissue can be determined by contacting a compound as defined herein wherein R₁ is a group comprising a fluorophore moiety, or a salt, solvate or stereoisomer thereof, with a sample comprising said cell or tissue and measuring the fluorescent signal emitted upon light excitation.

Compounds of any of the above formulae of the invention where R1 is a warhead such as AOMK comprise groups that react with the active site in the target enzyme (Caspase-1), thereby providing irreversible inhibition effects. Therefore, compounds of the invention wherein R₁ is a group can be used in the prevention or treatment of diseases or disorders in which Caspase-1 activity is involved.

Accordingly, in another aspect, the invention is directed to a compound of the invention wherein R₁ is a warhead such as AOMK, or a salt, solvate or stereoisomer thereof, for use as a medicament.

In another aspect, the invention is directed to a compound of the invention where R1 is a warhead such as AOMK, or a salt, solvate or stereoisomer thereof, for use in the prevention and/or treatment of a disease or disorder associated with an increased Caspase-1 activation selected from:
Autoinflammatory diseases including cryopyrin-associated periodic syndromes, Muckle-Wells syndrome, Schnitzler's syndrome, familial Mediterranean fever, macrophage activation syndrome, Crohn's disease, ulcerative colitis, and inflammatory bowel disease.
Ischemic diseases and injuries including atherosclerosis, myocardial infarction, heart ischemia, kidney ischemia, neonatal brain ischemia, hypoxia-ischemia injuries, stroke-like brain injuries, and ischemic optic neuropathy.
Neurodegenerative and neurological diseases including Alzheimer's disease, Parkinson's disease, Huntington's disease, mild cognitive impairment, amyotrophic lateral sclerosis (ALS), multiple sclerosis, multiple system atrophy, epilepsy, Creutzfeldt-Jakob disease, and adrenoleukodystrophy.
Metabolic and endocrine diseases including type 2 diabetes, dyslipidemias, obesity, metabolic syndrome, metabolic-dysfunction-associated fatty liver disease (MAFLD), metabolic dysfunction-associated steatohepatitis (MASH), cirrhosis, autoimmune hepatitis, primary sclerosing cholangitis and alpha-1 antitrypsin deficiency.
Inflammatory and autoimmune diseases including osteoarthritis, rheumatoid arthritis, psoriatic arthritis, gout, lupus nephritis, Behçet disease, and sterile inflammatory conditions such as syndromes triggered by aluminum salts.
Pulmonary diseases including steroid-resistant asthma, chronic obstructive pulmonary disease (COPD), idiopathic pulmonary fibrosis, and acute lung injury.
Ophthalmic diseases including retinal detachment, macular degeneration, diabetic retinopathy, retinitis pigmentosa, blunt ocular injury, glaucoma, and non-arteritic ischemic optic neuropathy.
Infectious and immune-related diseases including bacterial infections (e.g., Brucella, Staphylococcus aureus, and Salmonella), viral infections (e.g., influenza, Maraba virus, vesicular stomatitis virus), and sepsis.
Skin and sensory conditions including inflammatory acne, plaque psoriasis, dermatitis, cytotoxicity-related skin damage, age-related hearing loss, and drug-induced hearing loss.
Oncological diseases including cancers such as non-small cell lung cancer, breast cancer, melanoma, renal cell carcinoma, and pancreatic cancer.
Other conditions including peritonitis, polycystic kidney disease, acute kidney injury, joint loosening, and traumatic brain injury.

In a preferred embodiment, the invention is directed to a compound of the invention where R1 is a warhead such as AOMK, or a salt, solvate or stereoisomer thereof, for use in the prevention and/or treatment of peritonitis, metabolic-dysfunction-associated fatty liver disease (MAFLD), metabolic dysfunction-associated steatohepatitis (MASH), cirrhosis, inflammatory bowel disease, Crohn disease, ulcerative colitis and idiopathic pulmonary fibrosis.

The term "treatment" or "to treat" in the context of this specification means administration of a compound or composition according to the invention to ameliorate or eliminate the disease or one or more symptoms associated with said disease. "Treatment" also encompasses ameliorating or eliminating the physiological sequelae of the disease.

The term "ameliorate" in the context of this invention is understood as meaning any improvement on the situation of the patient treated.

The term "prevention" or "to prevent" in the context of this specification means administration of a compound or composition according to the invention to reduce the risk of acquiring or developing the disease or one or more symptoms associated with said disease.

In an embodiment, any method or use described herein can further comprise administering to the patient at least one other therapeutic agent.

The present invention is additionally explained below by means of examples. This explanation must by no means be interpreted as a limitation of the scope of the invention as it is defined in the claims.

### EXAMPLES

### Example 1. Synthetic route of Ac-Trp (5-F)-Arg-Ser-Asp-AOMK

### Synthesis of Warhead:

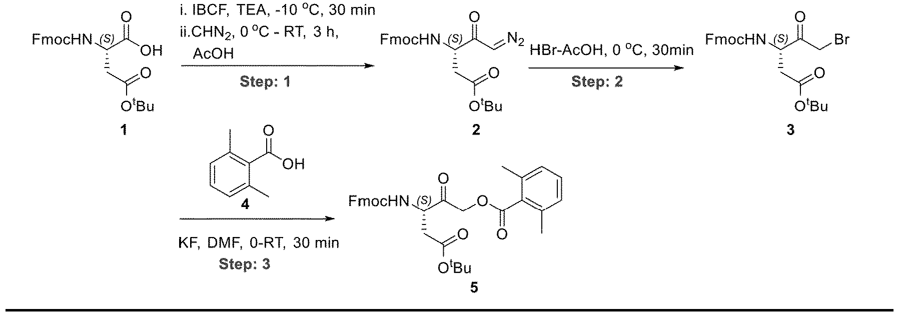

### Synthetic scheme for Ac-Trp (5-F)-Arg-Ser-Asp-AOMK:

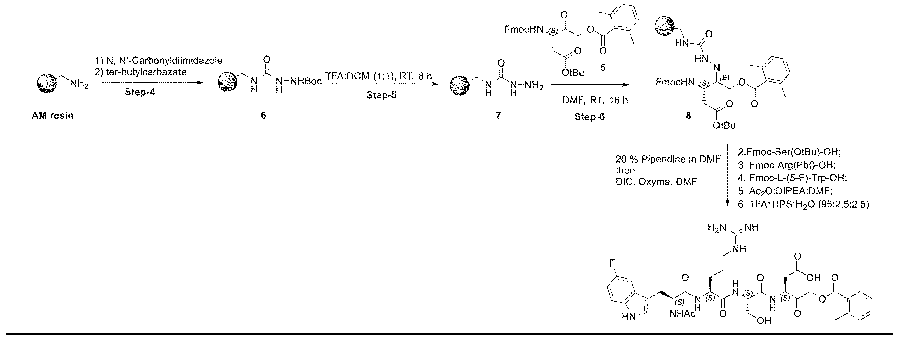

### Example 2. Results with Ac-Trp (5-F)-Arg-Ser-Asp-AOMK

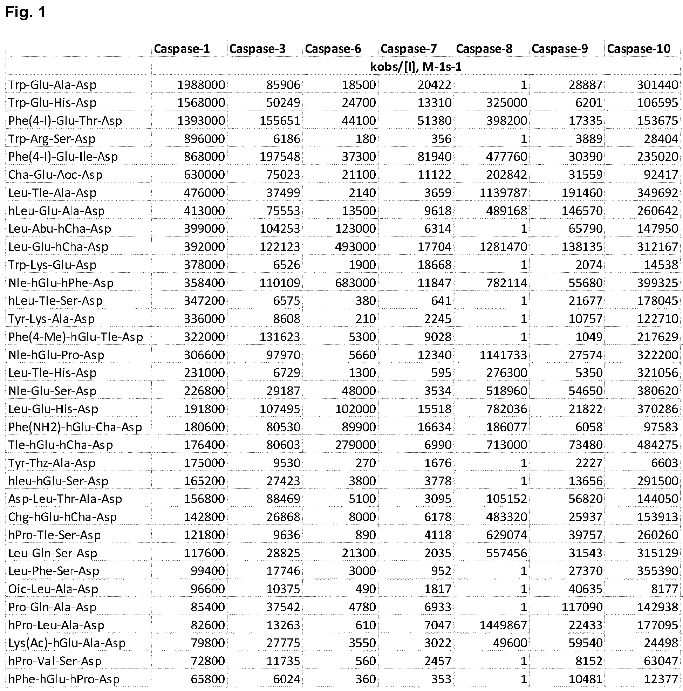

Figure 1 shows inhibitors potency of CASP1, CASP3, CASP6, CASP7, CASP8, CASP9 and CASP10 inhibition (expressed as Kobs/I, irreversible inhibition constant) in recombinant protein.

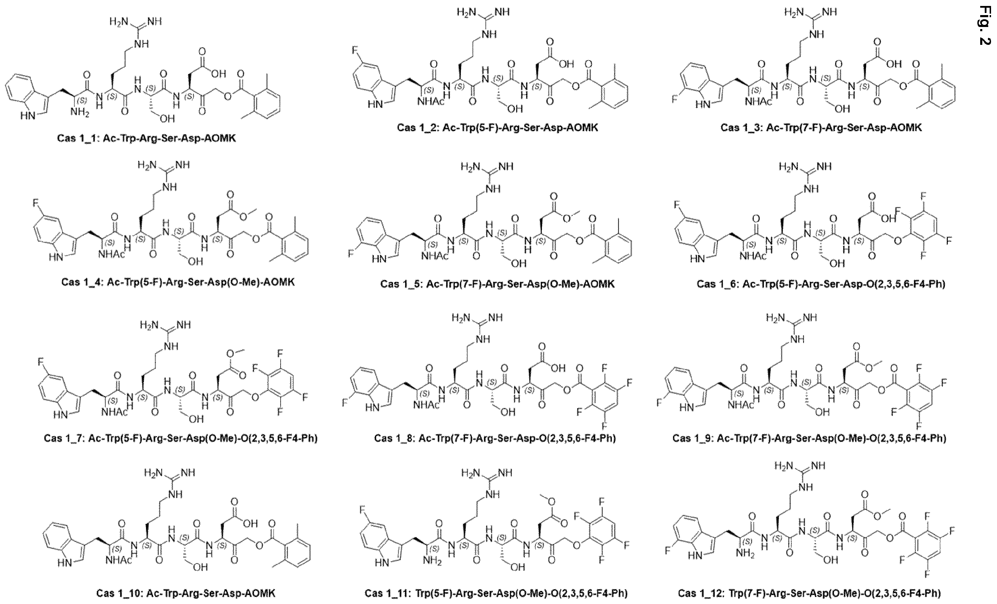

Figure 2 shows the structures of the ADME lead optimization sublibrary from sequence Trp-Arg-Ser-Asp.

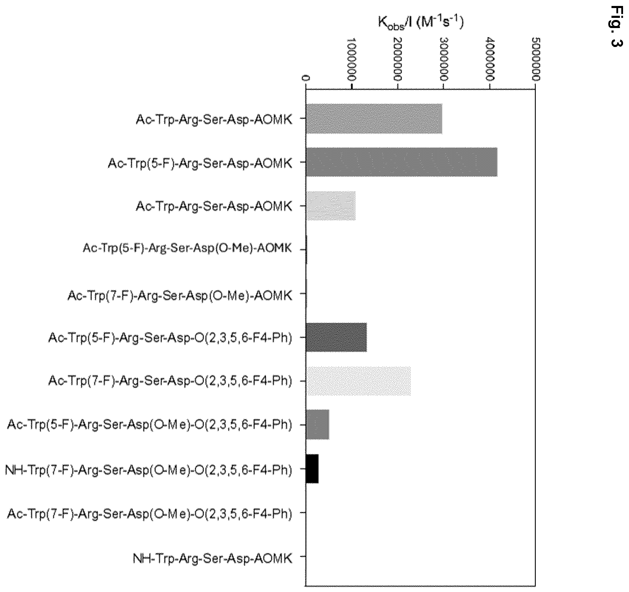

Figure 3 shows inhibitors potency of CASP1 inhibition (expressed as Kobs/I, irreversible inhibition constant) in recombinant protein.

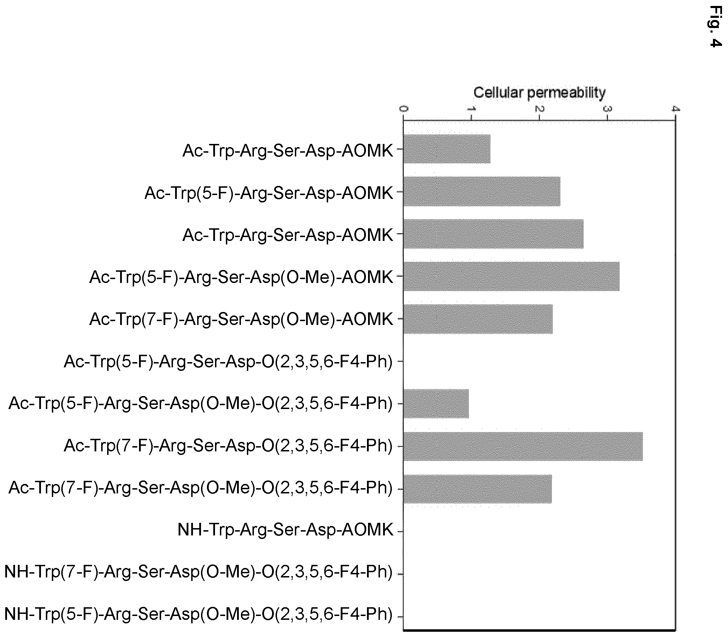

Figure 4 shows Apical to basal cellular permeability (expressed as *Papp (10⁻⁶ cm*/*sec*)) in Caco-2 cells.

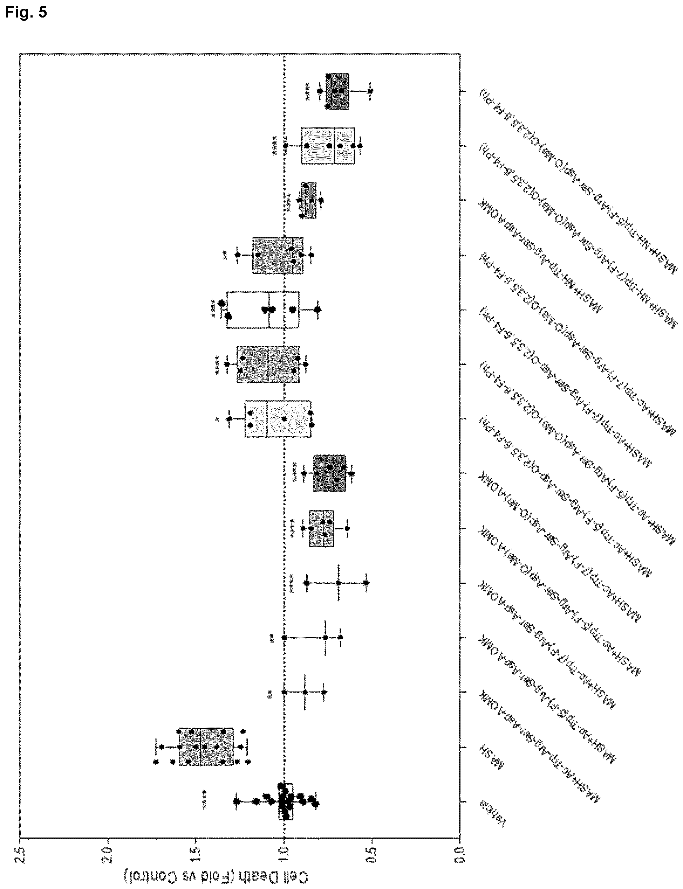

Figure 5 shows cellular death (expressed as an increase with respect to vehicle) in murine primary hepatocytes treated with Vehicle, MASH conditions metabolic stimuli (glucose, insulin and fatty acids) and inflammatory/pro-apoptotic (TNF-α, IL-1β and TGF-β) plus the caspase-1 inhibitors at 0.5µM for 24h. * p<0.05 vs MASH, **** p<0.001 vs MASH.

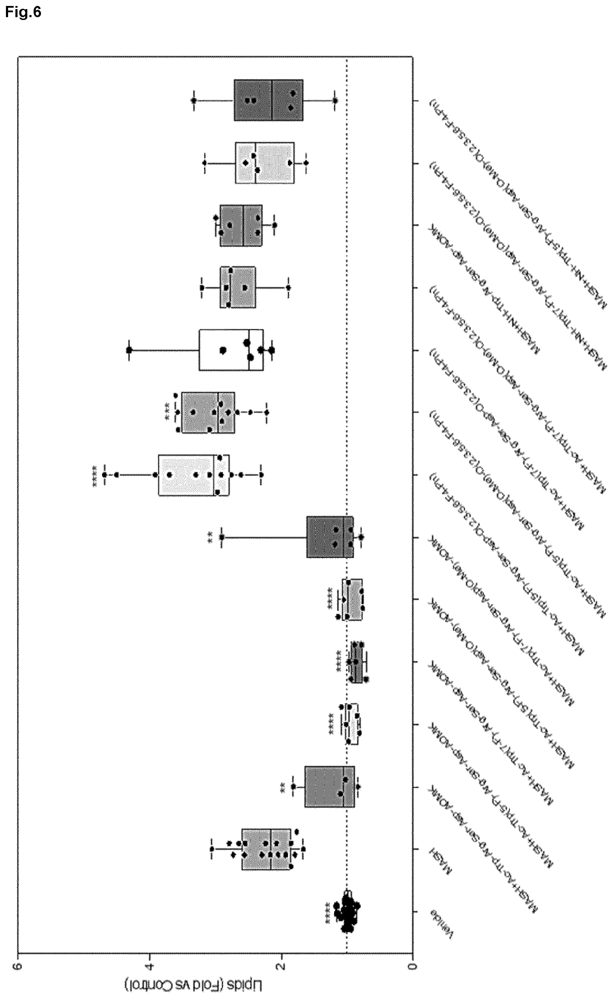

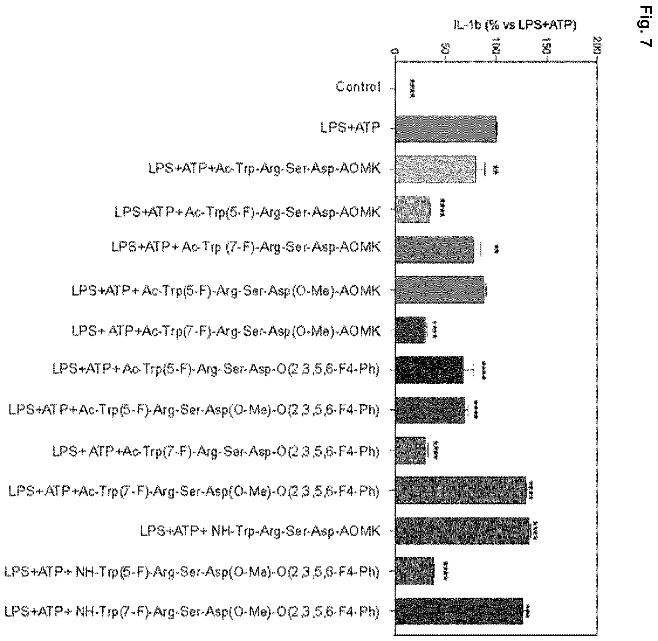

Figure 6 shows lipid accumulation (expressed as an increase with respect to vehicle) in murine primary hepatocytes treated with Vehicle, MASH conditions metabolic stimuli (glucose, insulin and fatty acids) and inflammatory/pro-apoptotic (TNF-α, IL-1β and TGF-β) plus the caspase-1 inhibitors at 0.5µM for 24h. * p<0.05 vs MASH, **** p<0.001 vs MASH. Figure 7 shows production of pro-inflammatory cytokine IL-1β (expressed as an increase compared to the control) in human primary macrophages treated with vehicle (Control), LPS+ATP, LPS+ATP plus the caspase-1 inhibitors at 0.1µM. *** p<0.01, **** p<0.001 vs LPS+ATP.

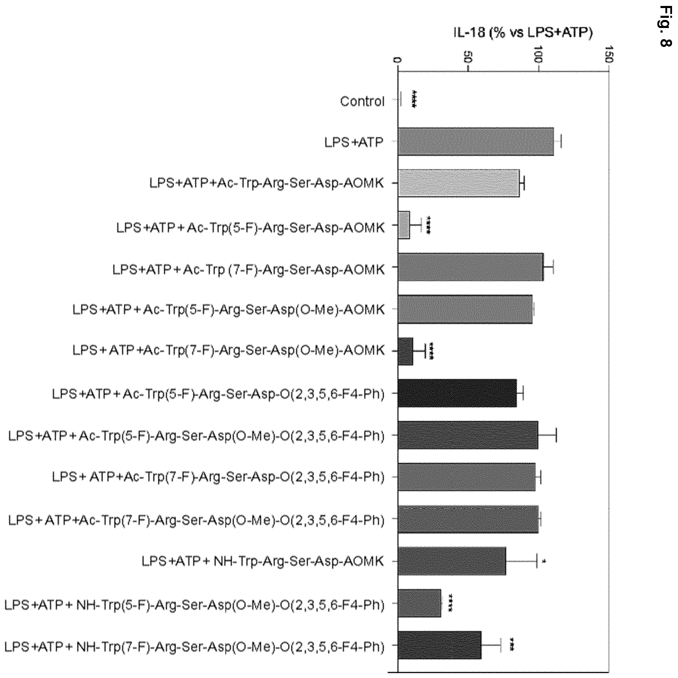

Figure 8 shows production of pro-inflammatory cytokine IL-18 (expressed as an increase compared to the control) in human primary macrophages treated with vehicle (Control), LPS+ATP, LPS+ATP plus the caspase-1 inhibitors at 0.1µM. *** p<0.01, **** p<0.001 vs LPS+ATP.

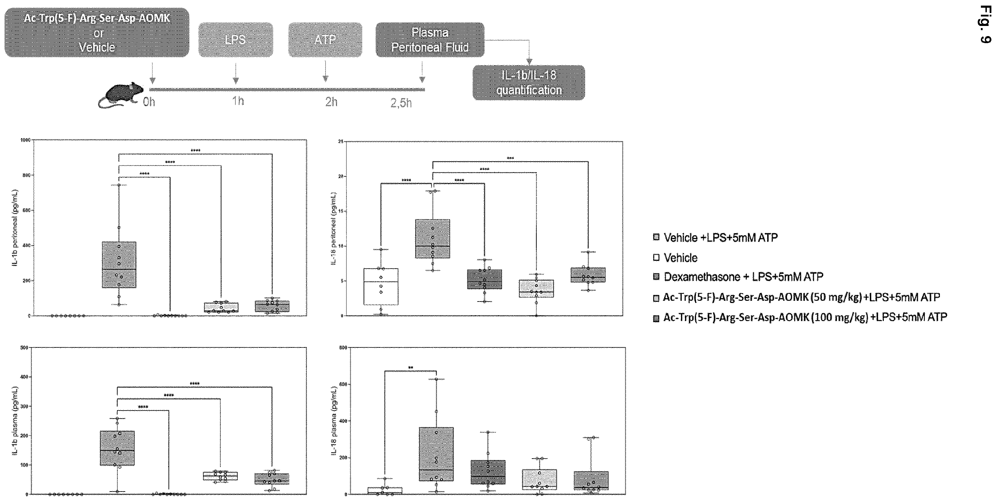

Figure 9. In vivo model or peritonitis and inflammasome activation. Inflammation was induced in mice by administration of LPS+ATP. Production of pro-inflammatory cytokines in peritoneal lavages and plasma. (A and B) IL-1β and IL-18 in peritoneal washings, (C and D) IL-1β and IL-18 in plasma. Control animals (vehicle), treated with LPS+ATP or LPS+ATP+KIN-124 at 50 mg/kg (50) and 100 mg/kg (100). * p<0.1 ** p<0.05*** p<0.01, **** p<0.001 vs LPS+ATP. n=10

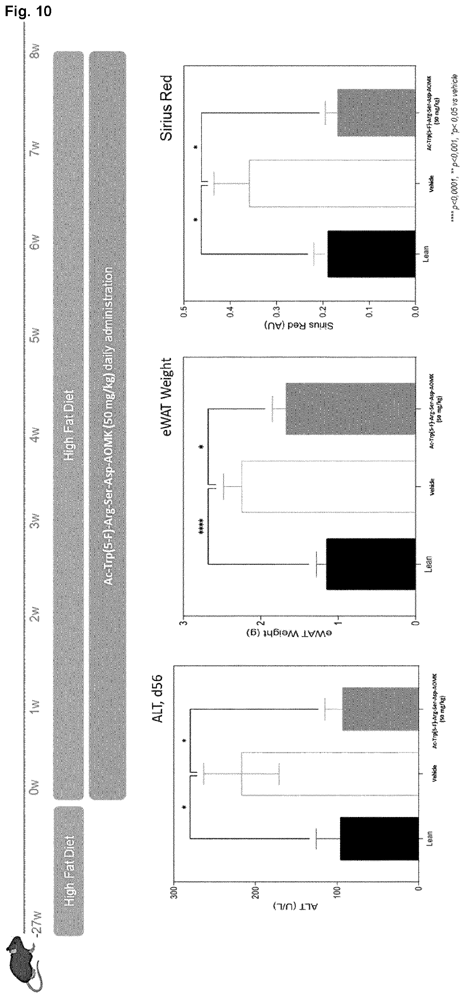

Figure 10. In vivo model of MASH. Diet-induced MASH mouse model treated with one of the KT caspase-1 inhibitors for 8 weeks (daily intraperitoneal administration) at 50 mg/kg. Effects on ALT, eWAT weight and Sirius Red staining in liver as a measurement of fibrosis. * p<0.05 vs vehicle, **** p<0.001 vs vehicle, n=15 animals/group. *ALT: alanine in micetransaminase; eWAT: epididymal white adipose tissue*

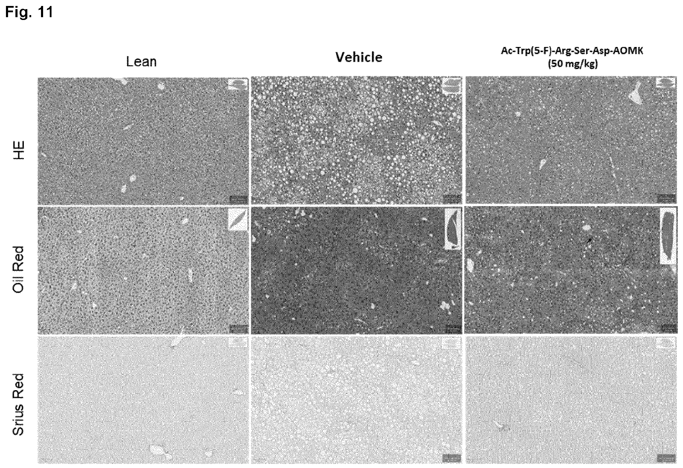

Figure 11. In vivo model of MASH: Liver histology. Diet-induced MASH mouse model treated with one of the KT caspase-1 inhibitors for 8 weeks (daily intraperitoneal administration) at 50 mg/kg. Representative images of H&E, Oil Red and Sirius Red stains in histological liver sections.

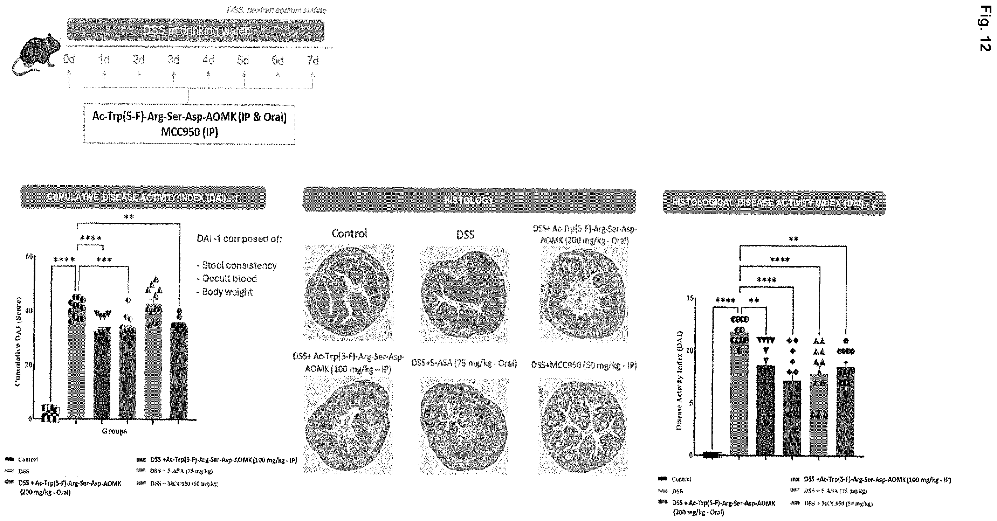

Figura 12. **In** vivo model of ulcerative colitis. Ulcerative colitis was induced in mice by daily oral administration of dextran sulfate (DSS) for 7 days. A. Effect of daily treatment with one of the KT caspase-1 inhibitors, 5-ASA (mesalazine) and MCC950 (inflammasome inhibitor) on cumulative disease activity index (DAI-1). DAI-1 was calculated with measurements of stool consistency, occult blood in heces and body weight. ** p<0.01, *** p<0.001 and **** p<0.0001 vs DSS. B. Representative images of colon histology, staining with H&E. C. Histological disease activity index (DAI-2). DAI-2 was calculated with measurements of severity and extent of inflammation, crypt damage, and percentage of involvement. ** p<0.01, and **** p<0.0001 vs DSS

### Example 3

**Table 1. Selectivity profiles for caspase -3,-6,-7,-8,-9 and -10. Expressed as caspase-1 kobs/[I] (M-1s-1) fold over other caspases kobs/[I], (M-1s-1)**

| | **Caspase-1 kobs/[I] (M-1s-1) fold over other caspases kobs/[I], (M-1s-1)** | | | | | |
|---|---|---|---|---|---|---|
| | **Caspase-3** | **Caspase-6** | **Caspase-7** | **Caspase-8** | **Caspase-9** | **Caspase-10** |
| Trp-Glu-Ala-Asp | 23,1416452 | 107,459459 | 97,3459994 | 1988000 | 68,8198844 | 6,59501062 |
| Trp-Glu-His-Asp | 31,2045555 | 63,4817814 | 117,806161 | 4,82461538 | 252,862442 | 14,7098832 |
| Phe(4-I)-Glu-Thr-Asp | 8,94952417 | 31,5873016 | 27,1117166 | 3,49824209 | 80,3576579 | 9,06458435 |
| Trp-Arg-Ser-Asp | 144,852547 | 4977,77778 | 2520,28747 | 896000 | 230,393417 | 31,5451305 |
| Phe(4-I)-Glu-Ile-Asp | 4,39386883 | 23,2707775 | 10,5931169 | 1,81681179 | 28,562027 | 3,6933027 |
| Cha-Glu-Aoc-Asp | 8,39742479 | 29,8578199 | 56,6444884 | 3,10586127 | 19,962926 | 6,81695221 |
| Leu-Tle-Ala-Asp | 12,6936944 | 222,429907 | 130,0845 | 0,41762201 | 2,48615899 | 1,36119915 |
| hLeu-Glu-Ala-Asp | 5,46633869 | 30,5925926 | 42,9403202 | 0,84429071 | 2,81776626 | 1,58455095 |
| Leu-Abu-hCha-Asp | 3,82722555 | 3,24390244 | 63,1889016 | 399000 | 6,06475148 | 2,69685705 |
| Leu-Glu-hCha-Asp | 3,20988207 | 0,79513185 | 22,1418888 | 0,30589869 | 2,8378036 | 1,25573946 |
| Trp-Lys-Glu-Asp | 57,9223553 | 198,947368 | 20,2485537 | 378000 | 182,23015 | 26,0002293 |
| Nle-hGlu-hPhe-Asp | 3,25495251 | 0,52474378 | 30,2523846 | 0,45824505 | 6,43678161 | 0,89751456 |
| hLeu-Tle-Ser-Asp | 52,8061823 | 913,684211 | 541,653666 | 347200 | 16,017346 | 1,9500688 |
| Tyr-Lys-Ala-Asp | 39,0315619 | 1600 | 149,660591 | 336000 | 31,2354746 | 2,73816315 |
| Phe(4-Me)-hGlu-Tle-Asp | 2,446376 | 60,754717 | 35,6668144 | 322000 | 306,959009 | 1,47958514 |
| Nle-hGlu-Pro-Asp | 3,12953797 | 54,1696113 | 24,8460292 | 0,26853906 | 11,1192509 | 0,95158287 |
| Leu-Tle-His-Asp | 34,3283765 | 177,692308 | 388,519991 | 0,83604777 | 43,1775701 | 0,71950164 |
| Nle-Glu-Ser-Asp | 7,77049228 | 4,725 | 64,1693074 | 0,4370279 | 4,15004575 | 0,5958699 |
| Leu-Glu-His-Asp | 1,78426461 | 1,88039216 | 12,3598402 | 0,24525739 | 8,78929521 | 0,5179784 |
| Phe(NH2)-hGlu-Cha-Asp | 2,24265048 | 2,00889878 | 10,8572803 | 0,97056818 | 29,8118191 | 1,85072588 |
| Tle-hGlu-hCha-Asp | 2,18851501 | 0,63225806 | 25,2360515 | 0,24740533 | 2,40065324 | 0,36425585 |
| Tyr-Thz-Ala-Asp | 18,3632303 | 648,148148 | 104,39036 | 175000 | 78,5986975 | 26,5017668 |
| hleu-hGlu-Ser-Asp | 6,02414471 | 43,4736842 | 43,7245249 | 165200 | 12,0972466 | 0,56672384 |
| Asp-Leu-Thr-Ala-Asp | 1,77236557 | 30,745098 | 50,6656327 | 1,49117468 | 2,75959169 | 1,08851093 |
| Chg-hGlu-hCha-Asp | 5,31496503 | 17,85 | 23,1150248 | 0,29545643 | 5,5056483 | 0,92779989 |
| hPro-Tle-Ser-Asp | 12,6406487 | 136,853933 | 29,5803381 | 0,19361783 | 3,06361144 | 0,46799354 |
| Leu-Gln-Ser-Asp | 4,0797324 | 5,52112676 | 57,7864261 | 0,21095835 | 3,72824398 | 0,37318101 |
| Leu-Phe-Ser-Asp | 5,6012848 | 33,1333333 | 104,389834 | 99400 | 3,63171355 | 0,27969273 |
| Oic-Leu-Ala-Asp | 9,31051749 | 197,142857 | 53,1598759 | 96600 | 2,37726098 | 11,8141052 |
| Pro-Gln-Ala-Asp | 2,27480618 | 17,8661088 | 12,3170471 | 85400 | 0,72935349 | 0,59746393 |
| hPro-Leu-Ala-Asp | 6,22801882 | 135,409836 | 11,7212998 | 0,05697076 | 3,68207551 | 0,46641633 |
| Lys(Ac)-hGlu-Ala-Asp | 2,87310317 | 22,4788732 | 26,4025092 | 1,60887097 | 1,34027545 | 3,25747525 |
| hPro-Val-Ser-Asp | 6,2036428 | 130 | 29,627218 | 72800 | 8,9306525 | 1,15469641 |
| hPhe-hGlu-hPro-Asp | 10,9237032 | 182,777778 | 186,396986 | 65800 | 6,27802691 | 5,3164557 |

## Claims

1. A compound of formula (I): 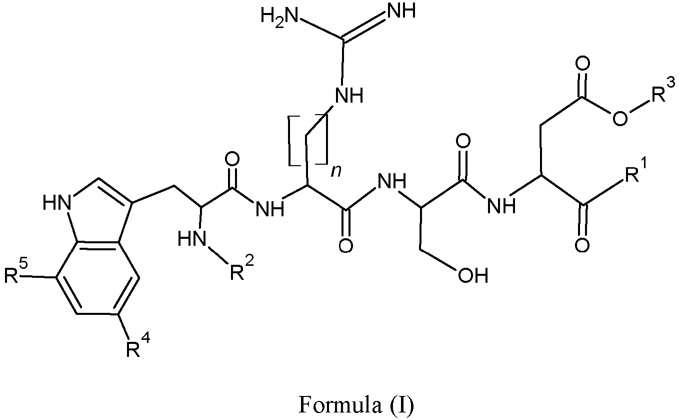
or a salt, solvate or stereoisomer thereof;
wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen;
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms;
n is an integer between 1 and 3; and
R₁ is a chemically reactive group such as a fluorophore or a warhead.

2. The compound of formula (I) of claim 1, wherein the compound is of formula (Ia): 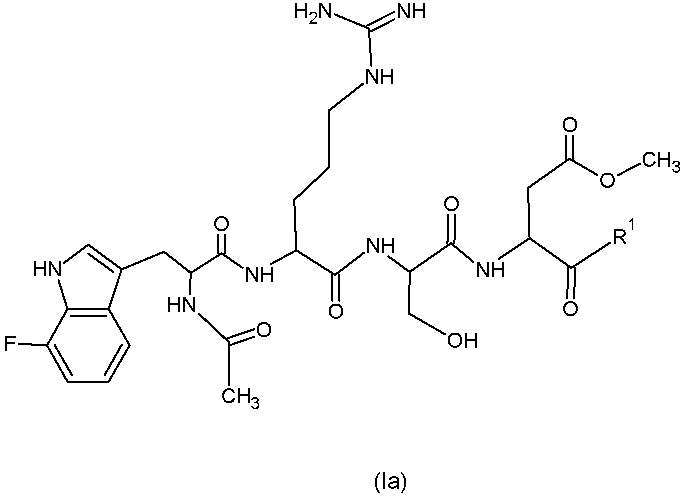 wherein the radicals are as defined in claim 1.

3. The compound of formula (I) of claim 1 or 2, wherein
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom; and
R₁ is a chemically reactive group as defined in claim 1.

4. The compound of formula (la) of claim 2, wherein the compound is of formula (II): 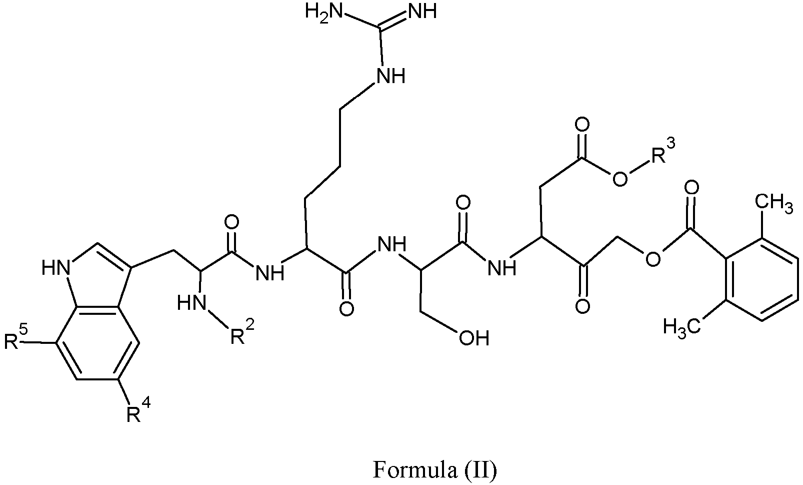 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms.

5. The compound of formula (II) of claim 4, wherein
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom.

6. The compound of formula (Ia) of claim 2, wherein the compound is of formula (III): 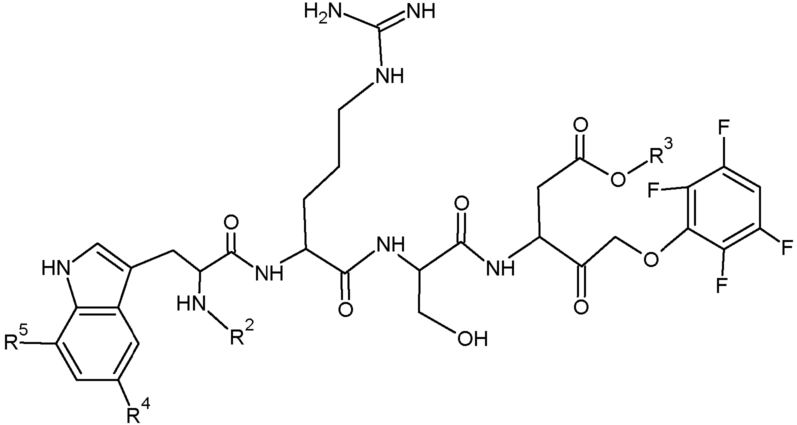 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms.

7. The compound of formula (III) of claim 6, wherein
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom.

8. The compound of formula (la) of claim 2, wherein the compound is of formula (IV): 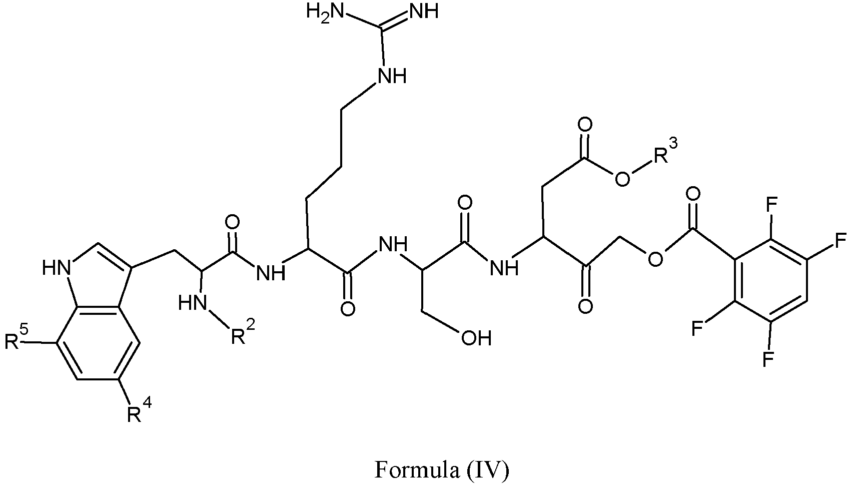 wherein:
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, a halogen or a C₁₋₄ alkyl including branched alkyl and cycloalkyl, wherein each C₁₋₄ alkyl is optionally substituted with 1 -3 halogen atoms.

9. The compound of formula (III) of claim 8, wherein
R₂ and R₃ are independently selected from a hydrogen, a C₁₋₄ alkyl including branched alkyl and cycloalkyl, and a -C(=O)-(C₁₋₄)alkyl, where R₂ is preferably an acetyl group and R₃ is hydrogen; and
R₄, and R₅ are each independently selected from a hydrogen, or a halogen atom.

10. The compound of any one of claims 1 to 9, wherein said compound is selected from any one of the following chemical compounds (protonated or deprotonated) or a salt, solvate or stereoisomer thereof:
a. 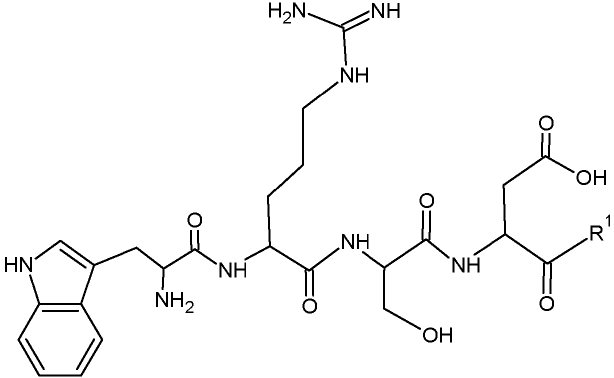
b. 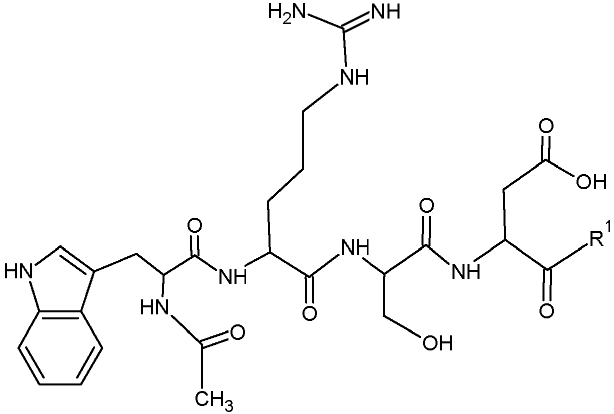
c. 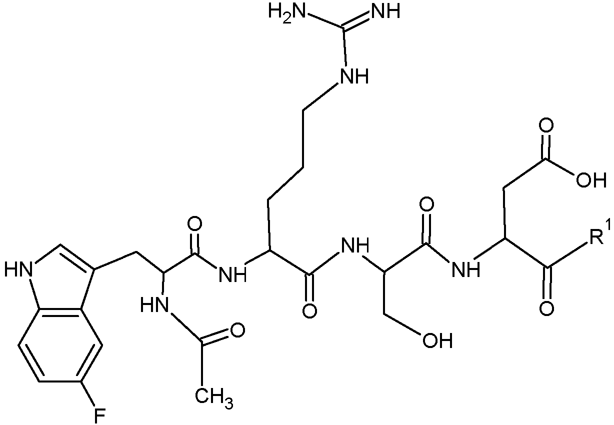
d. 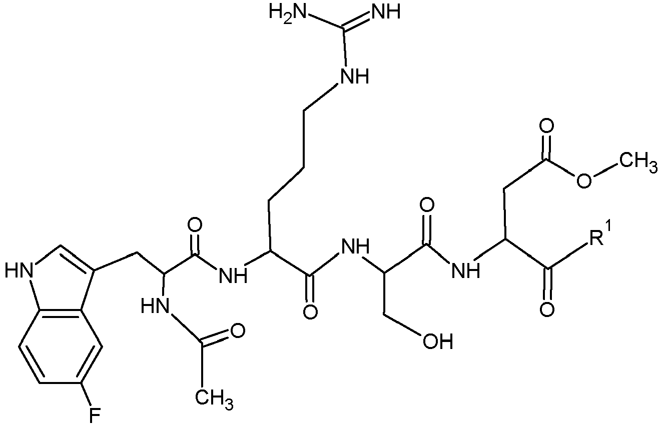
e. 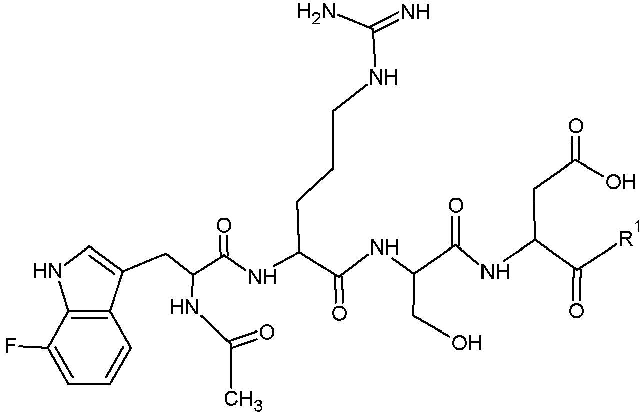
f. 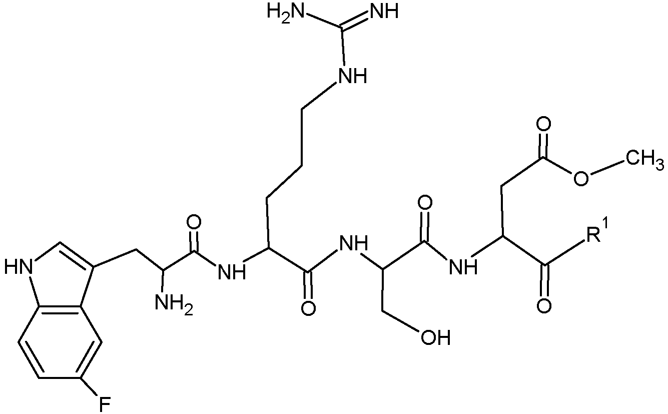
g. 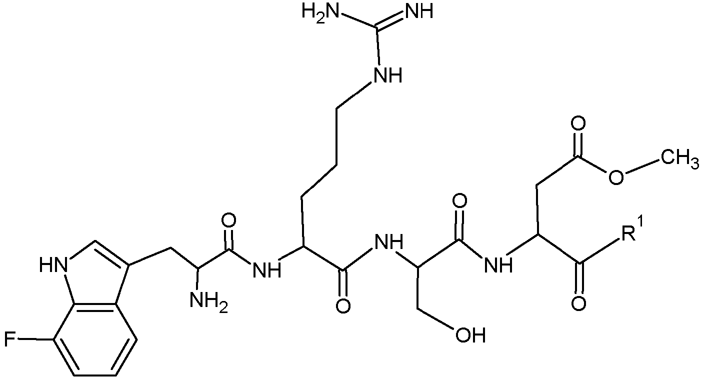
h.

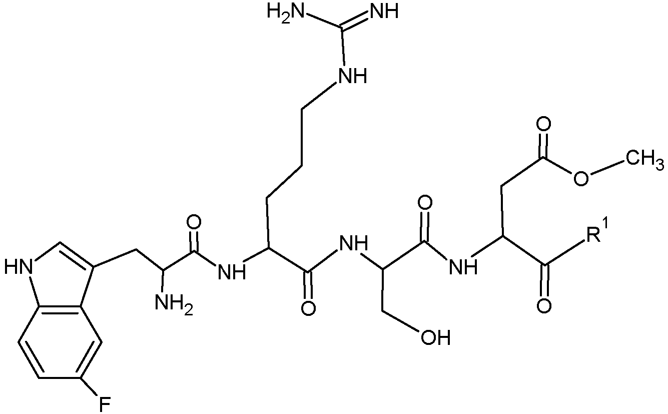

11. The compound according to claim 10, wherein R1 is a warhead (to be use in the context of inhibitors) selected from the group consisting of: 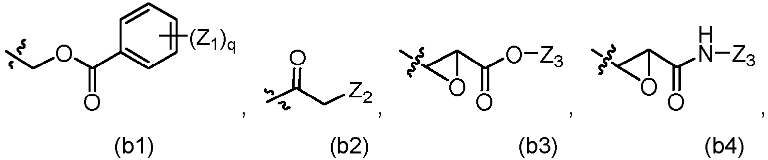 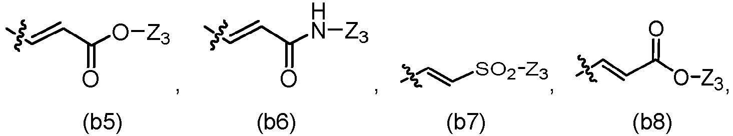 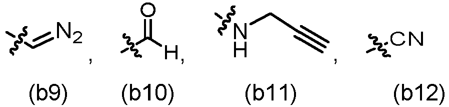 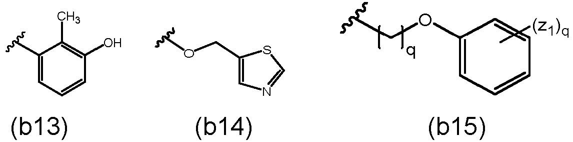 wherein
q is 0, 1, 2, 3, 4 or 5,
each Z₁ is independently selected from C₁₋₆ alkyl, halogen, -CN, -NO₂ and C₁₋₆ alkoxyl,
Z₂ is halogen, and
Z₃ is selected from C₁₋₆ alkyl, C₆₋₁₀ aryl and (C₆₋₁₀)aryl(C₁₋₆)alkyl.

12. The compound according to claim 10, wherein R1 is a warhead having the following formula:

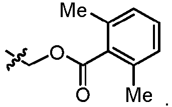

13. The compound according to claim 10, wherein R1 is a warhead having the following formula: 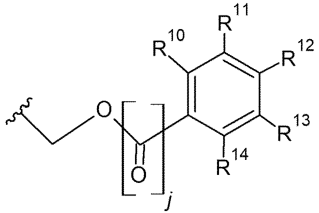 wherein
j is 0 or 1;
R₁₀, R₁₁, R₁₂, R₁₃ and R₁₄ are each independently selected from hydrogen, halogen or a C₁₋₄ alkyl including branched alkyl and cycloalkyl, phenyl, phenoxy, nitro, nitrile, PhCO, NHAc, wherein each alkyl is optionally substituted with 1 -3 halogen atoms.

14. The compound of any one of claim 1 to 13, wherein said compound is selected from any one of the following chemical compounds (protonated or deprotonated) or a salt, solvate or stereoisomer thereof:

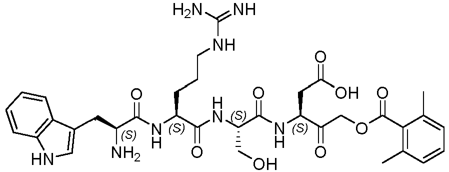

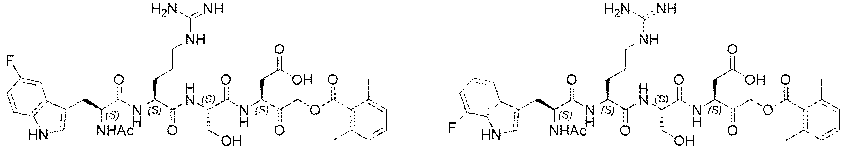

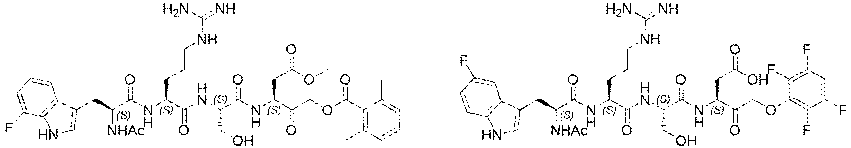

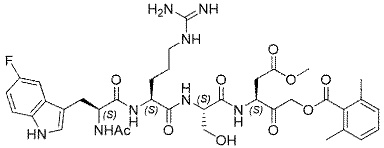

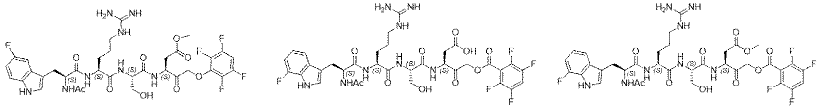

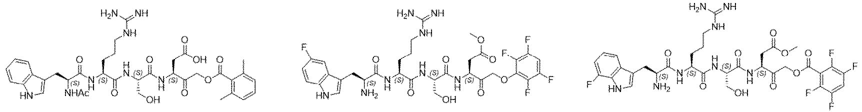

15. The compound of any one of claim 1 to 14, wherein said compound is the following chemical compound (protonated or deprotonated) or a salt, solvate or stereoisomer thereof:

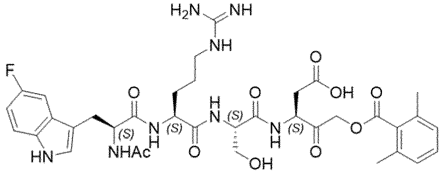

16. Compound according to any of the precedent claims, wherein all the amino acids in the compound are in the L configuration.

17. Pharmaceutical composition comprising a compound according to any one of claims 1 to 16 and optionally a pharmaceutically acceptable excipient.

18. Compound according to any one of claims 1 to 16 or composition according to claim 17, for use as a medicament.

19. Compound according to any one of claims 1 to 16 or composition according to claim 17, for use in the prevention and/or treatment of a disease selected from the group consisting of peritonitis, cryopyrin-associated periodic syndromes, Muckle-Wells syndrome, Schnitzler's syndrome, familial Mediterranean fever, macrophage activation syndrome, Crohn's disease, ulcerative colitis, inflammatory bowel disease, atherosclerosis, myocardial infarction, heart ischemia, kidney ischemia, neonatal brain ischemia, hypoxia-ischemia injuries, stroke-like brain injuries, ischemic optic neuropathy, Alzheimer's disease, Parkinson's disease, Huntington's disease, mild cognitive impairment, amyotrophic lateral sclerosis (ALS), multiple sclerosis, multiple system atrophy, epilepsy, Creutzfeldt-Jakob disease, adrenoleukodystrophy, type 2 diabetes, dyslipidemias, obesity, metabolic syndrome, metabolic-dysfunction-associated fatty liver disease (MAFLD), metabolic dysfunction-associated steatohepatitis (MASH), cirrhosis, autoimmune hepatitis, primary sclerosing cholangitis alpha-1 antitrypsin deficiency, osteoarthritis, rheumatoid arthritis, psoriatic arthritis, gout, **lupus nephritis,** Behçet disease, sterile inflammatory conditions such as syndromes triggered by aluminum salts, steroid-resistant asthma, chronic obstructive pulmonary disease (COPD), idiopathic pulmonary fibrosis, acute lung injury, retinal detachment, macular degeneration, diabetic retinopathy, retinitis pigmentosa, blunt ocular injury, glaucoma, non-arteritic ischemic optic neuropathy, bacterial infections (e.g., Brucella, Staphylococcus aureus, and Salmonella), viral infections (e.g., influenza, Maraba virus, vesicular stomatitis virus), sepsis, inflammatory acne, plaque psoriasis, dermatitis, cytotoxicity-related skin damage, age-related hearing loss, drug-induced hearing loss, cancer, non-small cell lung cancer, breast cancer, melanoma, renal cell carcinoma, pancreatic cancer, polycystic kidney disease, acute kidney injury, joint loosening, and traumatic brain injury.

20. Compound according to any one of claims 1 to 16 or composition according to claim 17, for use in the prevention and/or treatment of a disease selected from the group consisting of: peritonitis, metabolic-dysfunction-associated fatty liver disease (MAFLD), metabolic dysfunction-associated steatohepatitis (MASH), cirrhosis, inflammatory bowel disease, Crohn disease, ulcerative colitis and idiopathic pulmonary fibrosis.
